(19) Europäisches Patentamt
European Patent Office
Office européen des brevets

(11) EP 4 260 850 A1

(12) EUROPEAN PATENT APPLICATION
published in accordance with Art. 153(4) EPC

(43) Date of publication:
**18.10.2023 Bulletin 2023/42**

(21) Application number: **21907063.8**

(22) Date of filing: **14.12.2021**

(51) International Patent Classification (IPC):
***A61K 31/122*** (2006.01) ***A61K 36/11*** (2006.01)
***A61P 25/00*** (2006.01) ***A23L 33/10*** (2016.01)

(52) Cooperative Patent Classification (CPC):
**A23L 33/10; A61K 31/122; A61K 31/704; A61K 36/11; A61P 25/00**

(86) International application number:
**PCT/KR2021/018989**

(87) International publication number:
**WO 2022/131759 (23.06.2022 Gazette 2022/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **14.12.2020 KR 20200174821**

(71) Applicants:
- **GHPharm Co., Ltd.**
  **Goyang-si, Gyeonggi-do 10497 (KR)**
- **Korea University Research and Business Foundation**
  **Seoul 02841 (KR)**

(72) Inventors:
- **PARK, Gil Hong**
  **Seoul 02841 (KR)**
- **KO, Jae Young**
  **Seoul 05505 (KR)**
- **KIM, Ha Na**
  **Seoul 05505 (KR)**
- **LEE, Eun Jae**
  **Seoul 05505 (KR)**

(74) Representative: **Schwarz & Partner Patentanwälte GmbH**
**Patentanwälte**
**Wipplingerstraße 30**
**1010 Wien (AT)**

# (54) PHARMACEUTICAL COMPOSITION COMPRISING PTEROSIN COMPOUND AND DERIVATIVE THEREOF AS ACTIVE INGREDIENT FOR PREVENTION OR TREATMENT OF PKA-RELATED DISEASE

(57) The present invention relates to a composition for preventing or treating diseases related to protein kinase A (PKA, cAMP-dependent protein kinase) comprising a pterosin compound and derivatives thereof as an active ingredient and, more specifically, to a composition for preventing or treating neuropsychiatric diseases comprising a pterosin compound defined by formula 1 or derivatives thereof as an active ingredient.



Processed by Luminess, 75001 PARIS (FR)

(Cont. next page)

FIG. 10

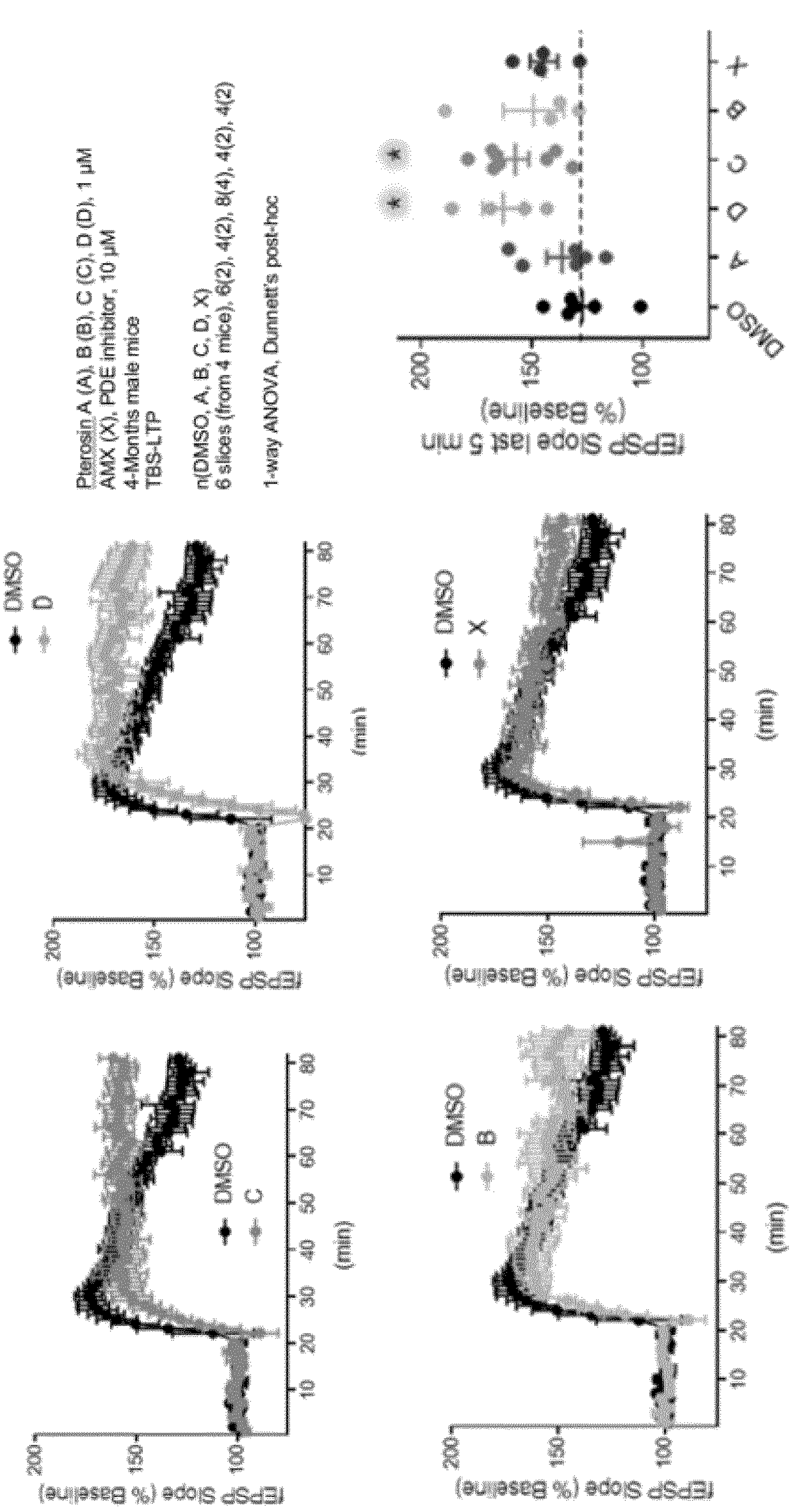
Pterosin A (A), B (B), C (C), D (D), 1 µM
AMX (X), PDE inhibitor, 10 µM
4-Months male mice
TBS-LTP
n(DMSO, A, B, C, D, X)
6 slices (from 4 mice), 6(2), 4(2), 8(4), 4(2), 4(2)
1-way ANOVA, Dunnett's post-hoc
fEPSP Slope last 5 min (% Baseline)
DMSO
A
D
C
B
X
fEPSP Slope (% Baseline)
(min)

## Description

### TECHNICAL FIELD

The present application claims priority to Korean Patent Application No. 2020-0174821 filed on December 14, 2020, and the entire specification thereof is incorporated herein by reference.

The present invention relates to a composition for preventing or treating diseases related to protein kinase A (PKA, cAMP-dependent protein kinase) comprising a pterosin compound and derivatives thereof as an active ingredient and, more specifically, to a composition for preventing or treating neuropsychiatric diseases comprising a pterosin compound defined by formula 1 or derivatives thereof as an active ingredient.

### BACKGROUND ART

Depression is a mood disorder that causes persistent feelings of sadness and loss of interest. It is estimated that more than 50% of patients with depression do not experience an adequate treatment response to known administered medications. As noted in an open-label study of medication treatment of depression, in most cases, 2 weeks or more of medication is required before significant improvement is observed (Rush et al, Am. J. Psychiatry 2006, 163: 1905). Since there is still no single effective treatment for depression and other related diseases, research thereon is ongoing.

Meanwhile, the use of psychotropic narcotic analgesic drugs and psychotropic drugs such as sleeping pills and anesthetics, and, among them, addictive drugs such as alcohol and heroin is increasing, and drug addiction is being recognized as a serious social problem. In order to treat this, drug addiction antidote has been actively developed. However, there are various problems such as high cost and side effects, and the effect is not remarkable.

On the other hand, protein kinase A (PKA, cAMP-dependent protein kinase) is a phosphorylation enzyme that covalently binds phosphate to a protein, and PKA is characterized in regulating its activity according to fluctuations in intracellular cAMP levels. Thus, this enzyme acts as the final effector of various hormones that work through the cAMP signaling pathway. In other words, PKA is essentially involved in all cellular responses due to the ultimate cAMP secondary transport system. PKA acts as a heterotetramer composed of two catalytic subunits with enzyme activities and two regulatory subunits having the function of regulating the activities. The catalytic subunits comprise the active functional area of the enzyme and also comprise an ATP binding domain, and a domain binding to the regulatory subunit. Regulatory subunits bind to each other in opposite directions to form homodimers, which have two cAMP-binding domains, a catalytic subunit-binding domain, and an 'autoinhibition' domain that can inhibit the catalytic domain. Thus, the activities of the catalytic subunits can be regulated. These regulatory subunits may also have other biological activities besides their role in regulating the catalytic subunits. PKA plays a role in fat cells, muscle cells comprising cardiomyocytes, hepatocytes, kidney cells, and nerve cells. The activities of the enzyme have different functions depending on the type of a cell.

cAMP response element-binding protein (CREB) as a major intracellular transcription factor binds to a specific DNA sequence called cAMP response elements (CRE) to increase or decrease the expression of subgenes. CREB was first reported in 1987 as a cAMP-responsive transcription factor regulating the somatostatin gene. ATP, which is the chemical energy of the cell, may be converted into cyclic AMP (cAMP) by adenylyl cyclase present in the membrane to regulate the activities of major proteins in the cell. CREB is mainly activated by Protein kinase A (PKA) activated by increase in cAMP intracellular concentration caused by external stimuli, and increases or inhibits the expression of genes related to intracellular nutrient supply and growth, physiological rhythm, spatial perception and memory.

PKA-CREB signaling is known to play an important role in neurodevelopment, synaptic and neuroplasticity, and neuroprotection, and it has been reported in many studies that PKA-CREB signaling is closely related to the treatment of various neuropsychiatric diseases such as depression.

### PRIOR ART DOCUMENT

### PATENT DOCUMENT

(Patent Document 0001) Korean Patent No. 10-2085358

### SUMMARY OF INVENTION

### TECHNICAL PROBLEM

Accordingly, the inventors of the present invention investigated the effects of a pterosin compound and derivatives thereof in bracken extract on a living body and confirmed that the pterosin compound and derivatives thereof could exhibit preventive or therapeutic effects on various neuropsychiatric diseases by activating the PKA-CREB signaling

pathway, and have completed the present invention.

Accordingly, an object of the present invention is to provide a pharmaceutical composition for the prevention or treatment of neuropsychiatric diseases, comprising a compound defined by the following formula 1, an isomer thereof, derivatives thereof, or a pharmaceutically acceptable salt thereof as an active ingredient.

In addition, an object of the present invention is to provide a pharmaceutical composition for the prevention or treatment of neuropsychiatric diseases, consisting of a compound defined by the following formula 1, an isomer thereof, derivatives thereof, or a pharmaceutically acceptable salt thereof.

In addition, an object of the present invention is to provide a pharmaceutical composition for the prevention or treatment of neuropsychiatric diseases, essentially consisting of a compound defined by the following formula 1, an isomer thereof, derivatives thereof, or a pharmaceutically acceptable salt thereof.

[Formula 1]

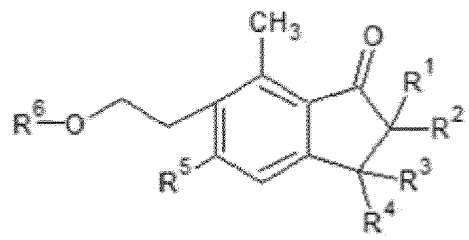

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

Another object of the present invention is to provide a food composition for the prevention or improvement of neuropsychiatric diseases, comprising a compound defined by the following formula 1, an isomer thereof, derivatives thereof, or a pharmaceutically acceptable salt thereof as an active ingredient.

Another object of the present invention is to provide a food composition for the prevention or improvement of neuropsychiatric diseases, consisting of a compound defined by the following formula 1, an isomer thereof, derivatives thereof, or a pharmaceutically acceptable salt thereof.

In addition, another object of the present invention is to provide a food composition for the prevention or improvement of neuropsychiatric diseases, essentially consisting of a compound defined by the following formula 1, an isomer thereof, derivatives thereof, or a pharmaceutically acceptable salt thereof.

[Formula 1]

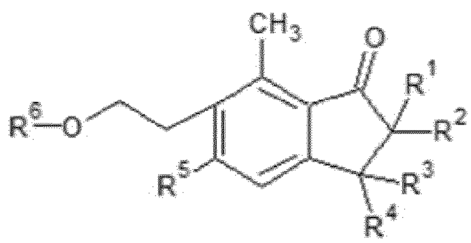

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

Another object of the present invention is to provide a use of a compound defined by the above-described formula 1, an isomer thereof, derivatives thereof, or a pharmaceutically acceptable salt thereof for preparing an agent for use in the treatment of neuropsychiatric diseases.

Another object of the present invention is to provide a method for treating neuropsychiatric diseases, comprising administering an effective amount of a composition comprising the compound defined by the above-described formula 1, an isomer thereof, or a pharmaceutically acceptable salt thereof to a subject in need thereof.

## SOLUTION TO PROBLEM

In order to achieve the above-described object of the present invention, the present invention provides a pharmaceutical composition for the prevention or treatment of neuropsychiatric diseases, comprising a compound defined by the following formula 1, an isomer thereof, derivatives thereof, or a pharmaceutically acceptable salt thereof as an active ingredient.

In addition, the present invention provides a pharmaceutical composition for the prevention or treatment of neuropsychiatric diseases, consisting of a compound defined by the following formula 1, an isomer thereof, derivatives thereof, or a pharmaceutically acceptable salt thereof.

In addition, the present invention provides a pharmaceutical composition for the prevention or treatment of neuropsychiatric diseases, essentially consisting of a compound defined by the following formula 1, an isomer thereof, derivatives thereof, or a pharmaceutically acceptable salt thereof.

[Formula 1]

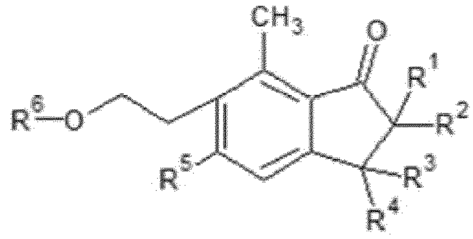

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

In order to achieve another object of the present invention, the present invention provides a food composition for the prevention or improvement of neuropsychiatric diseases, comprising a compound defined by the following formula 1, an isomer thereof, derivatives thereof, or a pharmaceutically acceptable salt thereof as an active ingredient.

In addition, the present invention provides a food composition for the prevention or improvement of neuropsychiatric diseases, consisting of a compound defined by the following formula 1, an isomer thereof, derivatives thereof, or a pharmaceutically acceptable salt thereof.

In addition, the present invention provides a food composition for the prevention or improvement of neuropsychiatric diseases, essentially consisting of a compound defined by the following formula 1, an isomer thereof, derivatives thereof, or a pharmaceutically acceptable salt thereof.

[Formula 1]

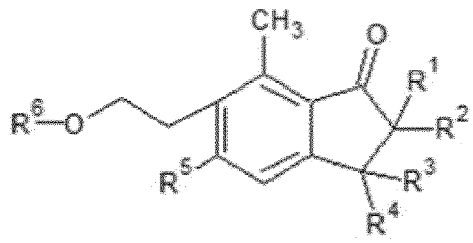

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

In order to achieve another object of the present invention, the present invention provides a use of a compound defined by the above-described formula 1, an isomer thereof, derivatives thereof, or a pharmaceutically acceptable salt thereof for preparing an agent for use in the treatment of neuropsychiatric diseases.

In order to achieve another object of the present invention, the present invention provides a method for treating neuropsychiatric diseases, comprising administering an effective amount of a composition comprising the compound defined by the above-described formula 1, an isomer thereof, or a pharmaceutically acceptable salt thereof to a subject in need thereof.

Hereinafter, the present invention will be described in detail.

The present invention provides a pharmaceutical composition for the prevention or treatment of neuropsychiatric diseases, comprising a compound defined by the following formula 1, an isomer thereof, derivatives thereof, or a pharmaceutically acceptable salt thereof as an active ingredient:

[Formula 1]

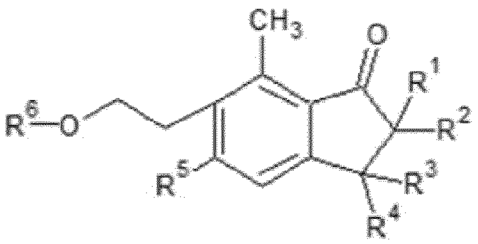

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

As used herein, the term "alkyl" is used to describe a group or part of a group comprising a straight-chain or branched-chain alkyl group containing 1 to 4 carbon atoms. Representative examples of such group comprise methyl, ethyl, propyl, isopropyl, n-butyl, isobutyl and tertbutyl.

As used herein, the term "alkenyl" is a straight-chain or branched monovalent or divalent hydrocarbon having 2 to 10 carbon atoms (e.g., C2-C10) and having one or more double bonds . Examples of alkenyl comprise ethenyl, propenyl, prophenylene, allyl and 1,4-butadienyl, but the present invention is not limited thereto.

As used herein, the term "alkynyl" is a straight-chain or branched monovalent or divalent hydrocarbon having 2 to 10 carbon atoms (e.g., C2-C10) and having one or more triple bonds . Examples of alkynyl comprise ethynyl, ethynylene, 1-propynyl, 1- and 2-butynyl and 1-methyl-2-butynyl, but the present invention is not limited thereto.

As used herein, the term "alcohol" is used to describe a straight-chain or branched-chain alcohol or alkoxy group having 1 to 4 carbon atoms. Such group comprises methanol, ethanol, propanol, butanol and their alkoxy groups. Examples of alkoxy groups comprise methoxy, ethoxy, isopropoxy, tert-butoxy. The term "alcohol having 1 to 4 carbon atoms" used in the present invention may be interpreted the same as "hydroxyalkyl having 1 to 4 carbon atoms."

In one aspect of the present invention, at least one of R1 to R4 in the above-described formula 1 may be OH or an alcohol having 1 to 4 carbon atoms.

In another aspect of the present invention, any one of R1 to R4 in the above-described formula 1 may be OH or an alcohol having 1 to 4 carbon atoms, and R6 may be hydrogen.

In a preferred aspect of the present invention, R3 and/or R4 in the above-described formula 1 may be OH.

In another preferred aspect of the present invention, R3 may be OH, and R4 may be hydrogen.

The 'compound defined by formula 1' of the present invention is a sesquiterpenoid present in bracken and may be a pterosin compound purified from bracken extract. In addition, the pterosin compound in the present invention may be pterosin A, pterosin B, pterosin C, pterosin D, pterosin J, pterosin M, pterosin N, pterosin P, pterosin S, pterosin Z, pteroside A, pteroside A2, pteroside B, pteroside C, pteroside D, pteroside N, pteroside P, pteroside Z, or sulfated pterosin C, and may preferably be A, pterosin C, pterosin D, protesin N, or an isomer thereof, but the present invention is not limited thereto.

Preferably, the compound defined by the above-described formula 1 in the present invention may be a pterosin compound containing a hydroxy (-OH) residue in R3 and/or R4, specifically pterosin A, C, and D or an isomer thereof.

Most preferably, the compound defined by the above-described formula 1 in the present invention may be pterosin C, D or an isomer thereof in which R3 and/or R4 are hydroxy (-OH).

In addition, the pterosin compound of the present invention may have a non-aromatic double bond and one or more asymmetric centers, which may occur as racemates and mixtures of racemates, single enantiomers, individual diastereomers, mixtures of diastereomers and cis- or trans-isomers, and may comprise all such isomeric forms.

The compound defined by the above-described formula 1 and derivatives thereof according to the present invention may be separated from nature or prepared by chemical synthesis of novel compounds known in the art. Preferably, the compound defined by the above-described formula 1 and derivatives thereof of the present invention may be separated and purified from natural plants . That is, they may be obtained from plants or part of plants by using conventional methods for extracting and separating substances. The stem, root or leaf may be appropriately dried and macerated or only dried and extracted by using an appropriate organic solvent to obtain a desired extract, and the desired extract may be purified by using a purification method known to those skilled in the art to which the present invention

belongs. Preferably, the compound defined by the above-described formula 1 and derivatives thereof of the present invention may be separated and purified from bracken.

The bracken may be, for example, Dennstaedtiaceae and Pteridaceae. Specific examples of such plant comprise Dennstaedtia scandens, Histiopteris incisa, Microlepia speluncae, Pteridium aquilinum var. Pteridium aquilinum var. latiusculum, Pteridium revolutum, Hypolepis punctata, Ceratopteris thalictroides, Pteris pauriea fauriei, Pteris dimidiata and Pteris ensiformi. These plants are distributed worldwide and may be found especially in Wulai Township, Taipei County and Mountain Datun, Taipei City, but the present invention is not limited thereto.

The compound defined by the above-described formula I and derivatives thereof may be separated and purified by a method comprising the following steps:

(a) extracting bracken by using water or an organic solvent having 1 to 4 carbon atoms;
(b) fractionating the extract obtained in step (a) by using ethyl-acetate; and
(c) separating and purifying the ethyl-acetate fraction obtained in step (b) by concentration gradient chromatography.

In step (a), the entire bracken plant or its dry body may be used, and the dried body of bracken may be pulverized with a grinder to increase the extraction efficiency. As a drying method, all of drying in the sun, drying in the shade, hot air drying, freeze drying and natural drying may be used. Preferably, hot air drying and freeze drying may be used.

The organic solvent having 1 to 4 carbon atoms used in the extraction of bracken may be methanol, ethanol, propanol, isopropanol, butanol, acetone, ether, chloroform, ethyl acetate, methylene chloride, hexane, cyclohexane, dichloromethane or petroleum ether. In one embodiment of the present invention, bracken was extracted by using water.

In step (b), the extract obtained in step (a) is fractionated, and distribution extraction is performed by using ethyl-acetate. The ethyl-acetate fraction obtained in step (b) is separated by gradient chromatography. As the chromatography, column chromatography and high-performance liquid chromatography (HPLC) filled with various synthetic resins such as silica gel and activated alumina may be used alone or in combination. Preferably, among the fractions obtained in step (b), the ethyl-acetate fraction is applied to a silica gel column, and the composition of the elution solvent is adjusted to gradually increase the polarity, thereby obtaining various fractions. Concentration gradient silica gel chromatography may be performed to adjust the composition of the elution solvent to gradually increase the polarity for a fraction having activities among the fractions obtained in the above-described process. However, the method for extracting and separating and purifying the compound is not necessarily limited to the above-described methods.

In step (c), the ethyl-acetate fraction obtained in step (b) is separated and purified by concentration gradient chromatography.

As the chromatography, column chromatography and high-performance liquid chromatography (HPLC) filled with various synthetic resins such as silica gel and activated alumina may be used alone or in combination. Preferably, novel compounds may be separated and purified by using high performance liquid chromatography.

The methods used to separate such compounds are well known in the art (see, e.g., [Takahashi et al, Phytother. Res, 2004, 18, 573, Sheridan et al, Planta Med., 1999, 65, 271, Nagao et al., Mutation Research, 1989, 215, 173, Murakami et al., Chem. Pharm. Bull, 1976, 24, 2241, and Kuraishi et al., Chem. Pharm. Bull, 1985, 33, 2305J). These compounds may also be prepared by chemical synthesis. Non-naturally occurring pterosin compounds may be converted from naturally occurring compounds (see, e.g., [Banerji er al, Tetrahedron Letters, 1974, 15, 1369, Hayashi et al., Tetrahedron Letters, 1991, 33, 2509, and McMorris et al., J.Org. Chem., 1992, 57, 6876]) or newly synthesized by methods well known in the art.

Synthetic chemical conversion and protecting group methods (protection and deprotection) useful for synthesizing the compound defined by the above-described formula 1 are known in the art (see, e.g., [R. Larock, Comprehensive Organic Transformations, VCH Publishers (1989) ; T. W. Greene and P. G. M. Wuts, Protective Groups in Organic Synthesis, 3rd Ed., John Wiley and Sons (1999); L. Fieser and M. Fieser, Fieser and Fieser's Reagents for Organic Synthesis, John Wileyand Sons (1994); and L. Paquette, ed., Encyclopedia of Reagents for Organic Synthesis, John Wiley and Sons (1995) and subsequent editions thereof]).

In one embodiment of the present invention, after 200 to 500 g of bracken was extracted with hot water, added with ethyl acetate (EA), mixed well and dried. The ethyl acetate extract was then dissolved in DMSO, diluted with water, and divided into 7 fractions by using column chromatography. The divided fractions were analyzed by HPLC to extract the compounds of the present invention (see Example 1).

In the present invention, the neuropsychiatric disease is a disease related to PKA and CREB signal transduction, and specific examples of the disease of the present invention may be any one or more selected from the group consisting of Huntington's disease, cerebral palsy, autism, attention deficit hyperactivity disorders (ADHD), paraplegia, depression, drug addiction, Rubinstein Taybi syndrome, insomnia, delayed sleep syndrome and advanced sleep phase syndrome, but the present invention is not limited thereto.

As described below, it is known through various references that PKA and CREB activation have preventive or therapeutic effects on the neuropsychiatric diseases described above.

**Depression, drug addiction, Rubinstein-Tayvey syndrome, cerebral palsy, autism, attention deficit hyperactivity disorder (ADHD) and paraplegia**

Abnormalities in a protein interacting with the KID domain of CREB, a CREB-binding protein (CBP), are associated with Rubinstein-Taybi syndrome, and several lines of evidence suggest that CREB dysfunction is associated with major depressive disorders (see [Belmaker, R. H.; Agam, Galila (2008) . "Major depressive disorder." New England Journal of Medicine. 358 (1): 55-68. doi:10.1056/nejmra073096. PMID 18172175]). Depressed mice with overexpression of CREB in the dentate gyrus behaved similarly to mice treated with antidepressants (see [Blendy, J. A. (2006). "The role of CREB in depression and antidepressant treatment." Biol Psychiatry. 59 (12): 1144-50. doi:10.1016/j.biopsych.2005.11.003. PMID 164577821). Postmortem examinations showed that the cortex of patients with untreated major depressive disorders contained reduced levels of CREB, as compared to both healthy controls and patients treated with antidepressants. The function of CREB may be regulated through a signaling pathway resulting from the binding of serotonin and noradrenaline to postsynaptic G-protein-coupled receptors. Dysfunction of such neurotransmitters is also associated with major depressive disorders. In addition, it is known that cerebral palsy, attention deficit hyperactivity disorder, and paraplegia may be improved by PKA and CREB activation.

**Insomnia, delayed sleep phase syndrome and advanced sleep phase syndrome**

Entrainment of the mammalian circadian clock is established through light induction of PER. Light excites melanopsin-containing photosensitive retinal ganglion cells that send signals to the suprachiasmatic nucleus (SCN) via the retinal hypothalamus (RHT). RHT signals the release of glutamate received by NMDA receptors in the SCN, resulting in calcium influx into the SCN. Calcium induces the activities of $Ca^{2+}$/calmodulin-dependent protein kinase, resulting in activation of PKA, PKC and CK2. Such kinases then phosphorylate CREB in a circadian manner further regulating downstream gene expression. Phosphorylated CREB recognizes cAMP response elements and acts as a transcription factor for Per1 and Per2 as two genes regulating the mammalian circadian clock. Such induction of the PER protein may accompany the circadian clock into the light-dark cycle and inhibit its own transcription through a transcription-translational feedback loop that may either advance or delay the circadian clock.

In addition, light pulses inducing phase changes during the night *in vivo* correlate with CREB phosphorylation in the SCN (see [Ginty, D. D.; Kornhauser, J. M.; Thompson, M. A.; Bading, H.; Mayo, K. E.; Takahashi, J. S.; Greenberg, M. E. (9 April 1993). "Regulation of CREB phosphorylation in the suprachiasmatic nucleus by light and a circadian clock." Science. 260 (5105): 238-241. Bibcode:1993Sci...260..238G. doi:10.1126/science.8097062. ISSN 0036-8075. PMID 8097062]). Experiments by Gunther Schutz in 2002 showed that mutant mice lacking the Ser142 phosphorylation site failed to induce the clock regulatory gene mPer1 in response to light pulses. Moreover, such mutant mice had difficulty accommodating light-dark cycles (see [Gau, Daniel; Lemberger, Thomas; von Gall, Charlotte; Kretz, Oliver; Le Minh, Nguyet; Gass, Peter; Schmid, Wolfgang; Schibler, Ueli; Korf, Horst W. (11 April 2002). "Phosphorylation of CREB Ser142 Regulates Light-Induced Phase Shifts of the Circadian Clock." Neuron. 34 (2): 245-253. doi:10.1016/S0896-6273(02)00656-6. PMID 11970866]).

The compound defined by the above-described formula 1 of the present invention comprises derivatives thereof, which comprise all pterosin derivatives derived from bracken. The compound comprises all pterosin derivatives by deletion, insertion, non-conservative or conservative substitution of chemical groups, or combinations thereof, as long as the compound has a PKA activating function by comprising wild type pterosin derivatives.

A derivative of pterosin refers to a chemical derivative that differs from the wild type in terms of one or more chemical groups. Insertion is usually performed with contiguous sequences of about 1 to 20 atoms, but larger insertion may be possible. Deletion is usually performed with about 1 to 30 atoms, but larger deletion may also be possible, for example, in some cases, one of the side chains may be deleted. Such derivatives may be prepared by chemical synthesis methods known in the art. Chemical group exchanges in chemical derivatives that do not entirely alter the activities of the molecule are known in the art. The most commonly occurring exchange is that between chemical groups.

In addition, the compound defined by the above-identified formula 1, in some cases, may be modified by phosphorylation, sulfation, acrylation, glycosylation, methylation, farnesylation and the like.

In the present invention, the derivative of the compound defined by the above-described formula 1 is a functional equivalent that exhibits the same biological activities as the natural compound, but may be a derivative whose properties are modified to maximize efficacy and minimize side effects, if necessary. Preferably, it is a compound in which the structural stability of the compound to heat, pH, etc. increases, the absorption rate in the body increases, or the physiological activities increase by mutation and modification of the side chain.

Derivatives of the compound defined by the above-described formula 1 may be obtained by extraction and purification from natural sources comprising bracken by methods well known in the art. Alternatively, since the structural formula of pterosin is known, the derivatives may be obtained by chemical synthesis. When prepared by chemical synthesis, the derivatives may be obtained by using a compound synthesis method widely known in the art.

In another embodiment of the present invention, the types and chemical structures of the compounds purified from bracken extract were identified by using nuclear magnetic resonance (NMR) (see Example 2).

In the present specification, the term "pharmaceutically acceptable salt" refers to a salt that is not toxic to cells or human beings exposed to the composition, and has a safety and efficacy profile suitable for administration to human beings.

The salt may be used in the form of either a pharmaceutically acceptable basic salt or acid salt. The basic salt may be used in the form of either an organic base salt or an inorganic base salt and selected from the group consisting of sodium salt, potassium salt, calcium salt, lithium salt, magnesium salt, cesium salt, aminium salt, ammonium salt, triethylaminium salt and pyridinium salt.

As an acid salt, an acid addition salt formed by a free acid is useful. Inorganic acids and organic acids may be used as free acids. Inorganic acids may comprise hydrochloric acid, hydrobromic acid, sulfuric acid, sulfurous acid, phosphoric acid, double phosphoric acid, and nitric acid. Organic acids may comprise citric acid, acetic acid, maleic acid, malic acid, fumaric acid, glucoic acid, methanesulfonic acid, benzenesulfonic acid, camphorsulfonic acid, oxalic acid, malonic acid, glutaric acid, acetic acid, glycolic acid, succinic acid, tartaric acid, 4-toluenesulfonic acid, galacturonic acid, embonic acid, glutamic acid, citric acid, aspartic acid, stearic acid, etc. However, the present invention is not limited thereto, and all salts formed by using various inorganic acids and organic acids commonly used in the art may be contained.

In addition, the compound defined by the above-described formula 1 may comprise, in addition to the above-described salt, all salts, hydrates, solvates, derivatives and the like that may be prepared by conventional methods. The addition salt may be prepared by a conventional method, and by dissolving in a water-miscible organic solvent such as acetone, methanol, ethanol, and acetonitrile, and adding an excessive amount of an organic base or adding an aqueous solution of an inorganic base, followed by precipitation or crystallization. Alternatively, the solvent or excess base may be evaporated from the mixture and then dried, or the precipitated salt may be suction filtered, to prepare an addition salt.

The pharmaceutical composition according to the present invention may comprise the compound defined by the above-described formula 1, an isomer thereof, derivatives thereof, or a pharmaceutically acceptable salt thereof alone or be formulated in a suitable form together with a pharmaceutically acceptable carrier, or further comprise excipients or diluents. In the above, the term 'pharmaceutically acceptable' refers to a non-toxic composition that is physiologically acceptable and does not cause an allergic reaction such as a gastrointestinal disorder, dizziness and a similar reaction, when administered to human beings.

A pharmaceutically acceptable carrier may further comprise, for example, a carrier for oral administration or a carrier for parenteral administration. The carrier for oral administration may comprise lactose, starch, cellulose derivatives, magnesium stearate, stearic acid and the like. In addition, various drug delivery materials used for oral administration of peptide preparations may be contained therein. In addition, the carrier for parenteral administration may comprise water, suitable oil, saline, aqueous glucose and glycol, and the like, and may further comprise a stabilizer and a preservative. Suitable stabilizers are antioxidants such as sodium bisulfite, sodium sulfite and ascorbic acid. Suitable preservatives are benzalkonium chloride, methyl- or propyl-paraben and chlorobutanol. The pharmaceutical composition of the present invention may further comprise, in addition to the above-described components, a lubricant, a wetting agent, a sweetening agent, a flavoring agent, an emulsifier, a suspending agent and the like. Reference may be made to other pharmaceutically acceptable carriers and agents known in the art.

The composition of the present invention may be administered to mammals comprising human beings by any method. For example, it may be administered orally or parenterally. Parenteral methods of administration may comprise intravenous, intramuscular, intraarterial, intramedullary, intrathecal, intracardiac, transdermal, subcutaneous, intraperitoneal, intranasal, enteral, topical, sublingual or rectal administration, but the present invention is not limited thereto.

The pharmaceutical composition of the present invention may be formulated into a preparation for oral administration or parenteral administration according to the administration route described above.

In the case of preparations for oral administration, the composition of the present invention may be formulated into powders, granules, tablets, pills, sugarcoated tablets, capsules, solutions, gels, syrups, slurries, suspensions, etc. by a method known in the art. For example, preparations for oral use may be obtained by combining the active ingredient with a solid excipient, milling same, adding suitable auxiliaries thereto, and processing same into a mixture of granules to obtain tablets or sugarcoated tablets. Examples of suitable excipients may comprise sugars (e.g., lactose, dextrose, sucrose, sorbitol, mannitol, xylitol, erythritol and maltitol), starches (e.g., corn starch, wheat starch, rice starch and potato starch), celluloses (e.g., cellulose, methyl cellulose, sodium carboxymethylcellulose and hydroxypropyl methyl-cellulose) and fillers (e.g., gelatin and polyvinylpyrrolidone). In addition, cross-linked polyvinylpyrrolidone, agar, alginic acid or sodium alginate may be added as a disintegrant, if desired. Furthermore, the pharmaceutical composition of the present invention may further comprise an anticoagulant, a lubricant, a wetting agent, a flavoring agent, an emulsifier and a preservative.

The preparation for parenteral administration may be formulated in the form of injections, creams, lotions, external ointments, oils, moisturizers, gels, aerosols and nasal inhalants by a method known in the art. Such formulations are described in prescriptions generally known to all pharmaceutical chemists.

The total effective amount of the composition of the present invention may be administered to the patient in a single dose or by a fractionated treatment protocol in which multiple doses are administered over a long period of time. The content of the active ingredient contained in the pharmaceutical composition of the present invention may vary depending on the severity of a disease. Preferably, the preferred total dose of the pharmaceutical composition of the present invention may be about 0.01 $\mu$g to 10,000 mg, most preferably 0.1 $\mu$g to 500 mg per kg of patient body weight per day. However, the effective dosage of the pharmaceutical composition for the patient is determined in consideration of various factors comprising, as well as the formulation method, administration route and number of treatments, the patient's age, weight, health condition, gender, severity of disease, diet and excretion rate. Therefore, considering them, those skilled in the art will be able to determine an appropriate effective dosage of the composition of the present invention. The pharmaceutical composition according to the present invention is not particularly limited in terms of its formulation, administration route and administration method, as long as it exhibits the effects of the present invention.

The present invention provides a food composition for the prevention or improvement of neuropsychiatric diseases, comprising a compound defined by the following formula 1, an isomer thereof, derivatives thereof, or a pharmaceutically acceptable salt thereof as an active ingredient.

[Formula 1]

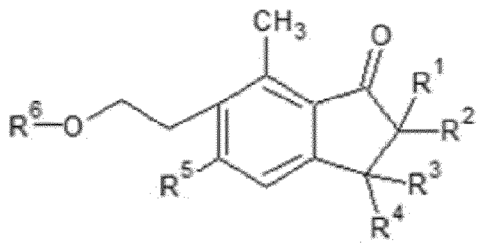

wherein

R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

The compound defined by the above-described formula 1 and the disease are as described above.

The food composition comprising the compound defined by the above-described formula 1 according to the present invention comprises all forms such as functional food, nutritional supplement, health food and food additives. The above-described types may be prepared in various forms according to conventional methods known in the art.

For example, as a health food, the food composition itself of the present invention may be prepared in the form of tea, juice or drink to be consumed, or consumed by granulation, encapsulation or powdering. In addition, the food composition of the present invention may be prepared in the form of a composition by mixing same with known substances or active ingredients known to have effects of reducing body fat, improving cholesterol and lowering a blood pressure.

In addition, functional foods may be prepared by adding the food composition of the present invention to beverages (e.g., alcoholic beverages), fruits and their processed foods (e.g., canned fruit, bottled fruit, jam and marmalade), fish, meat, and their processed foods (e.g., ham, sausage and corned beef), breads and noodles (e.g., udon, buckwheat noodles, ramen, spaghetti and macaroni), fruit juice, various drinks, cookies, taffy, dairy products (e.g., butter and cheese), edible vegetable oil, margarine, vegetable protein, retort food, frozen food and various seasonings (e.g., soybean paste, soy sauce and sauce).

The preferable content of the composition for food according to the present invention is preferably 0.01 to 50% by weight based on the total weight of the finally prepared food, but the present invention is not limited thereto. In order to use the food composition of the present invention in the form of a food additive, the food composition may be prepared and used in the form of a powder or concentrate.

In addition, the food composition of the present invention may comprise various flavoring agents or natural carbohydrates as additional components like conventional beverages. The carbohydrates comprise monosaccharides such as glucose and fructose, disaccharides such as maltose and sucrose, polysaccharides such as dextrins and cyclodextrins, and a sugar alcohol such as xylitol, sorbitol and erythritol. Examples of sweeteners comprise natural sweeteners such as thaumatin and stevia extract, and synthetic sweeteners such as saccharin and aspartame. The ratio of the natural carbohydrate may be generally about 0.01 to 0.04g, preferably about 0.02 to 0.03g per 100mL of the composition of the present invention, but the present invention is not limited thereto.

Definitions of terms for the excipients, binders, disintegrants, lubricants, corrigents, flavoring agents, etc. of the present invention are described in the references known in the art, and comprise those having the same or similar functions.

In another embodiment of the present invention, NIH3T3 cells as normal cells, and B16F10 cells as cancer cells were cultured in a 96-well plate, and Compounds 2 to 11 were then added to the cell medium, cultured and treated with MTT, and the optical density was measured at 550 nm with a microplate reader. LD50 values were calculated based on the measured results. As a result, it was found that the LD50 value of each compound was high in cancer cells and normal cells. As such, it was confirmed that each compound had low cytotoxicity (see Example 3).

In addition, the present invention provides a use of a compound defined by the following formula 1, an isomer thereof, or a pharmaceutically acceptable salt thereof for preparing a preparation for the treatment of neuropsychiatric diseases:

[Formula 1]

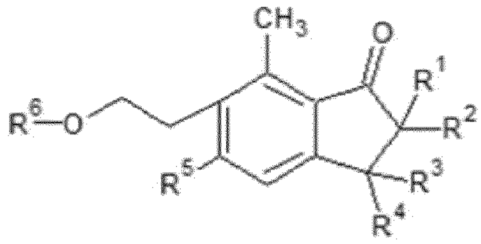

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

In addition, the present invention provides a method for treating neuropsychiatric diseases, comprising administering an effective amount of a composition comprising the compound defined by the following formula 1, an isomer thereof, or a pharmaceutically acceptable salt thereof to a subject in need thereof:

[Formula 1]

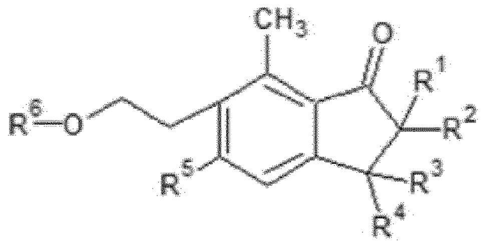

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

The 'effective amount' of the present invention refers to an amount that exhibits an effect of improving, treating, preventing, detecting, diagnosing, or suppressing or reducing a neuropsychiatric disease when administered to a subject, and the 'subject' refers to an animal, preferably may be mammals, especially animals comprising human beings, and may be cells, tissues, organs, etc. derived from animals. The subject may be a patient in need of the effect.

The 'treatment' of the present invention comprehensively refers to improving a neuropsychiatric disease or symptoms of a neuropsychiatric disease, which may comprise curing, substantially preventing, or improving the condition of a neuropsychiatric disease, and alleviating, curing or preventing one or most of the symptoms resulting from the disease, but the present invention is not limited thereto.

As used herein, the term "comprising" is used in the same sense as "including" or "characterized by." The composition or method according to the present invention does not exclude additional components or method steps not specifically mentioned. In addition, the term "consisting of" refers to excluding additional elements, steps or components not separately described. The term "essentially consisting of" means that, in addition to the described materials or steps, materials or steps that do not substantially affect the basic characteristics thereof may be contained in the scope of a composition or method.

## ADVANTAGEOUS EFFECTS OF INVENTION

As described above, the composition comprising the compound provided by the present invention, an isomer thereof or a pharmaceutically acceptable salt thereof as an active ingredient exhibits the effect of activating PKA and CREB and, thus, may be used to develop a therapeutic agent of the diseases selected from the group consisting of Huntington's disease, cerebral palsy, autism, attention deficit hyperactivity disorder (ADHD), paraplegia, depression, drug addiction, Rubinstein Taybi syndrome, insomnia, delayed sleep syndrome and advanced sleep phase syndrome, or a food for improving the treatment of the diseases.

## BRIEF DESCRIPTION OF DRAWINGS

FIG. 1 is a diagram illustrating a process of separating and purifying a pterosin compound and derivatives thereof.
FIG. 2 shows the results of confirming the effect of pterosin compounds and derivatives thereof on CREB phosphorylation in neurons and astrocytes, which are connective tissue cells of the brain.
FIG. 3 shows the regulatory subunit and the catalytic subunit of PKA.
FIG. 4 shows the 3D structure of the regulatory subunit of PKA.
FIG. 5 shows a binding model between pterosin A and CBD1.
FIG. 6 shows a binding model between pterosin A and CBD2.
FIG. 7 shows surface models of pterosin A, CBD1 and CBD2.
FIG. 8 shows the results of confirming the effects of pterosin A, C, D, and B on CREB, brain-derived neurotrophic factor (BDNF), and trk phosphorylation in neurons through Western blotting.
FIGS. 9 and 10 are the results of observing electrophysiological changes after treating a mouse brain tissue with a pterosin compound.
FIG. 11 is a graph showing the quantification and observation of the effects of promoting proliferation, differentiation and regeneration of nerve cells (Neuron) after treatment with pterosin D under a microscope.

## MODE FOR INVENTION

Hereinafter, the present invention will be described in detail by the following embodiments.
However, the following embodiments are only for illustrating the present invention, and the present invention is not limited thereto.

### Experimental methods

### Experimental apparatuses

1 H NMR and 13 C NMR spectra were measured by using deuterium chloroform (CDCl3) and dimethyl sulfoxide (DMSO-d 6) with 1 H NMR at 400 MHz and 13 C NMR at 100 MHz on a JEOL JUM ECP-400 spectrometer (Jeol, Tokyo, Japan).
Column chromatography was performed with silica gel (70-230 mesh; Merk, Darmstadt, Germany), RP-18 (40-63 mm; Merk) and Sephadex LH-20 (20-100 mm; Sigma, St. Louis, MO, USA). Thin layer chromatography (TLC) was performed by being pre-coated with Kiesel gel 60 F254 plates (0.25 mm; Merck) and 25 RP-18 F 254s plates (5 -10 cm, Merk). Spray reagent was 50% $H_2SO_4$. All chemicals and solvents used in column chromatography were of reagent grade and were purchased and used as received.

### MTT assay

NIH3T3 cells, as a mouse fibroblast cell line, and B16F10 cells, as a mouse melanoma cell line, were dispensed into a 96 well plate at $2\times10^3$ cells/well, and cultured in DMEM supplemented with 10% fetal bovine serum (FBS) at 5% $CO_2$ and 37°C. Compounds (pterosin A, pterosin B, pterosin C, pterosin D, pterosin P, pterosin Z, pteroside A, pteroside B and pteroside Z) were treated at different concentrations and then cultured for 48 hours. Thereafter, MTT solution (5 mg/ml) was added to each well at a concentration of 10 μg/well and incubated at 37°C for 4 hours. 100 μl of DMSO was added to each well, and the supernatant was removed. After incubation for 10 minutes, optical density was measured at 550 nm by using a Microplate Reader (Molecular Devices, Spectra Max, USA). An optical density is an index representing the number of surviving cells and is calculated by the formula below, and reproducibility was confirmed through three experiments.

Cell proliferation rate (%) = OD550 (sample) / OD550 (control) X 100

## Western blot analysis

Western blot analysis was performed by using the following method. Cells were lysed in RIPA buffer (20 mM Tris-Cl pH 7.4, 1 mM EDTA, 150 mM NaCl, 1% Triton X-100, 1 mM EGTA, 1 μM Na3VO4, 2.5 mM sodium pyrophosphate, 1 μM phenylmethylsulfonyl fluoride, 1 ug/ml leupeptin). The protein concentration was calculated by using BCA Protein Assay Reagent (BioRad, Hercules, CA, USA). Protein samples were separated by SDS-PAGE and transferred to polyvinylidene difluoride (PVDF) membranes (Millipore, Bedford, MA, USA). Membranes were incubated overnight at 4 °C with primary antibodies followed by HRP-conjugated goat anti-rabbit IgG (1:5,000; Pierce; Carlsbad, CA, USA). The used primary antibodies were actin (sigma; 1:1000), p-CREB (Cell signaling; 1:1000), tubulin (Cell signaling; 1:1000), p-PKA (Cell signaling; 1:1000), p -trk (Cell signaling; 1:1000) and BDNF (Abcam; 1:1000). All membranes were visualized by using a UVP Autochemi Darkroom Imaging System (Utra-Violet Products Ltd. UK) and Immobilon Crescendo Western HRP Substrate or Luminata Forte Western HRP Substrate (Millipore, USA).

## Electrophysiology experiments

1. The brain of a 4-month-old mouse was placed on an ice-cold dissection buffer, and a 300 μm-thick sagittal hippocampal slice was prepared by using a vibratome (dissection buffer: contents in mM: sucrose, 25 $NaHCO_3$, 5 KCl, 1.25 $NaH_2PO_4$, 0.5 $CaCl_2$, 3.5 $MgSO_4$, 10 D-glucose, 1.25 L-ascorbic acid and 2 Na-pyruvate bubbled with 95% $O_2$/5% $CO_2$).
2. Slices were recovered at 32°C for 1 hour in normal ACSF (ACSF: mM: 125 NaCl, 2.5 KCl, 1.25 $NaH_2PO_4$, 25 $NaHCO_3$, 10 glucose, 2.5 $CaCl_2$ and 1.3 $MgCl_2$ oxygen in 95% $O_2$/5% $CO_2$).
3. During recording, the slices were moved to a chamber submerged in ACSF at 28°C, and ACSF saturated with 95% $O_2$/5% $CO_2$ was continuously perfused (2ml/min). A Micropipette for stimulation and recording was fabricated by using boron acid glass capillaries (Harvard Instruments) and a micropipette electrode puller.
4. For input/output, paired-pulse ratio, and long-term potentiation (LTP) experiments, an ACSF-filled pipette (resistance: 1 MS2) was used to test the hippocampus. A field excitatory post-synaptic potential (fEPSP) was measured in the CA1 region. The fEPSP signal was amplified by using Multiclamp 700B (Molecular Devices) and digitized by using Digidata 1440A (Molecular Devices). The Schaffer collateral pathway was stimulated with a pipette filled with ACSF (0.3-0.5 MS2) every 20 seconds (LTP) or 15 seconds (input/output, paired-pulse ratio) .
5. After a stable baseline signal was acquired to induce LTP, theta-burst stimulation (40 trains of pulse, each train is composed with 4 stimulus in 100 Hz; 40 trains are divided by 4 burst, each containing 10 trains with Electrical stimulation of 1 sec inter-burst-interval; Within the 10 trains, there were 170 ms time inter-train-interval) was applied thereto. The pair-pulse ratio was measured after giving the pair stimulation at intervals of 25, 50, 75, 100, 200, and 300 ms .
6. To analyze the pharmacological effects of drug A (pterosin A) /C (pterosin C) /X (Drug X, amx, a phosphodiesterase (PDE) inhibitor) on synaptic properties, each drug was dissolved in DMSO at a stock concentration of 1000 times, and applied so that the concentrations in ACSF were 10 μM (X) and 1 μM (A/C), respectively. The same volume of DMSO as the experimental drug was applied as a control drug.

## Example 1: Purification of a pterosin compound and derivatives thereof

The bracken hot-water extract was extracted by using ethyl acetate and separated into 7 fractions, and pterosin compounds were separated from each fraction by using HPLC.

Specifically, 200 to 250 g of bracken collected in Gapyeong-gun, Gyeonggi-do, was washed, put in a steaming container (OSK-2002, Dr. Red Ginseng, Well sosanaTM, Daewoong Pharmaceutical), added with 1.5 L of water, steamed for 24 hours, and then additionally added with 3.5 L of water and aged for 72 hours, and the refrigerated mixture was used as a hot water extract.

After adding an equal volume of ethyl acetate (EA) to the hot water extract and mixing well, the EA layer was dried with a rotary evaporator and used as an EA extract.

The EA extract was dissolved in a minimum amount of DMSO and then diluted by using water. At this time, the dilution factor was diluted to 70% of the original volume of the hot water extract, assuming a yield of approximately 70%.

The EA extract was divided into 7 fractions under the conditions of chloroform ($CHCl_3$) and methanol by using silica gel column chromatography. Then, each fraction was analyzed by Prep-HPLC, and Compounds 2 to 11 could be extracted from fractions 2, 4, 5, 6, and 7. The process of extracting each compound is shown in FIG. 1.

**Example 2: Identification of the pterosin compounds of Compound 2-11 and derivatives thereof**

In order to identify each compound purified in Example 1, a single compound was separated from the EA fraction, and the type and chemical structure of the compound was then identified by the nuclear magnetic resonance (NMR) method described in the experimental method. The structure and name of each compound identified in the bracken extract are shown in Table 1 below, and specific NMR analysis results are shown in Table 2 below.

[Table 1]

| The name of the compounds | | |
|---|---|---|
| No. | Structure | Name |
| 2 | 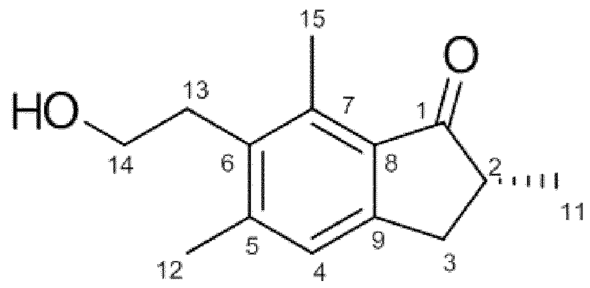 | (2R)-pterosin B |
| 3 | 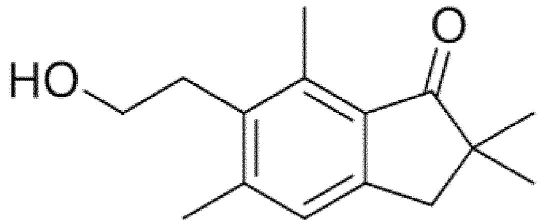 | pterosin Z |
| 4 | 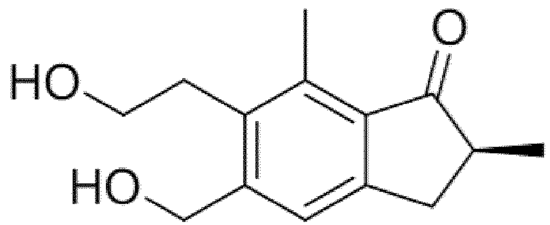 | (2S)-pterosin P |
| 5 | 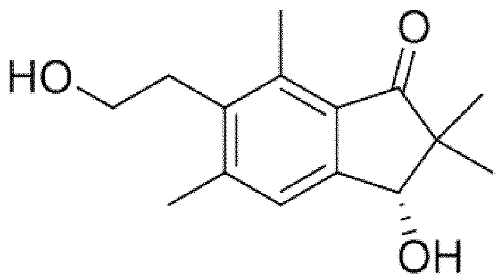 | (3R)-pterosin D |

(continued)

| The name of the compounds | | |
|---|---|---|
| No. | Structure | Name |
| 6 | 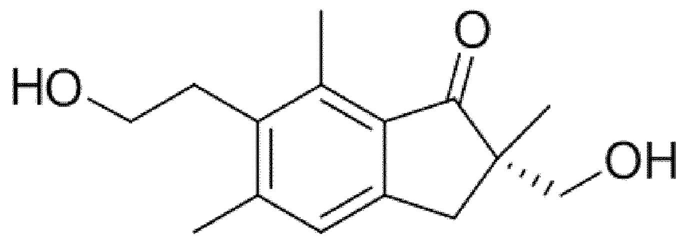 | (2S)-pterosin A |
| 7 | 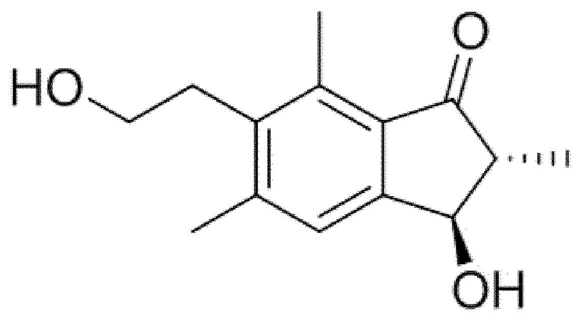 | (2R,3R)-pterosin C |
| 8 | 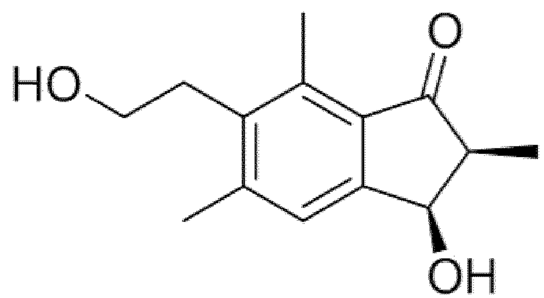 | (2S,3R)-pterosin C |

(continued)

| The name of the compounds | | |
|---|---|---|
| No. | Structure | Name |
| 9 | 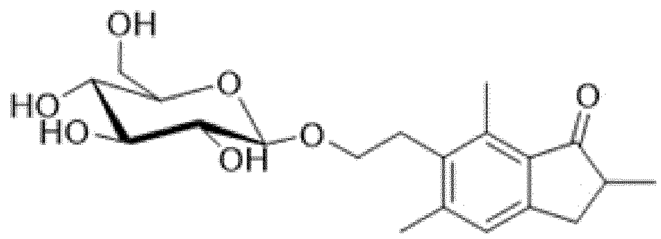 | (2R)-pteroside B |
| 10 | 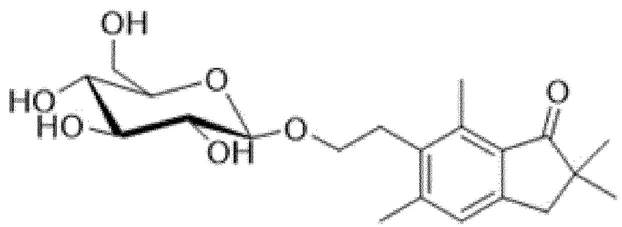 | pteroside Z |
| 11 | 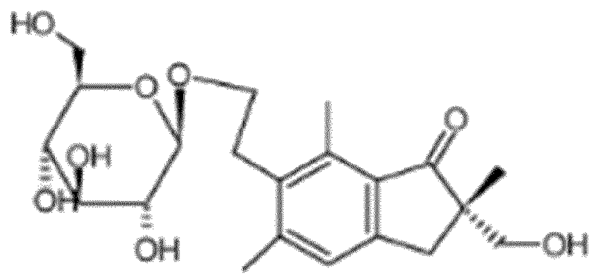 | (2S)-pteroside A |
| 12 | 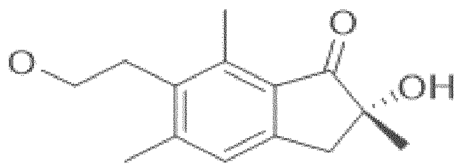 | pteroside N |

[Table 2]

**$^{13}$C-NMR Data ($CD_3OD$, 175 MHz)[a] of Compounds 2 to 11**

| | 2[b] | 3 | 4 | 5 | 6 | 7 | 8 | 9 | 10 | 11 |
|---|---|---|---|---|---|---|---|---|---|---|
| 1 | 210.6 s[c] | 212.3 s | 212.8 s | 211.8 s | 213.0 s | 210.5 s | 207.8 s | 212.7 s | 214.7 s | 2130 s |
| 2 | 42.5 d | 45.6 s | 44.1 d | 52.6 s | 52.8 s | 49.8 d | 54.9 d | 43.9 d | 46.8 s | 52.8 s |
| 3 | 33.8 t | 44.7 t | 35.1 t | 77.7 d | 37.7 t | 70.4 d | 76.0 d | 34.9 t | 42.7 t | 37.6 t |
| 4 | 125.7 d | 125.9d | 124.5 d | 126.3 d | 127.1 d | 126.9 d | 125.8 d | 127.0 d | 127.1 d | 127.1 d |
| 5 | 144.5 s | 144.4 s | 148.6 s | 146.5 s | 146.3 s | 146.4 s | 146.4 s | 146.4 s | 146.6 s | 146.5 s |
| 6 | 134.9 s | 134.8 s | 135.9 s | 138.5 s | 136.5 s | 138.7 s | 138.4 s | 136.4 s | 136.5 s | 136.3 s |
| 7 | 137.8 s | 138.4 s | 139.1 s | 138.6 s | 138.9 s | 138.2 s | 138.0 s | 139.0 s | 139.4 s | 139.1 s |
| 8 | 132.0 s | 131.1s | 134.0 s | 131.3s | 133.3 s | 132.4 s | 132.6 s | 133.0 s | 132.0 s | 133.2 s |
| 9 | 152.6s | 151.4s | 154.6 s | 153.9s | 154.2 s | 155.3 s | 154.7 s | 154.6 s | 153.2 s | 154.3 s |
| 10 | | 25.6 q | | 20.9 q | 21.4 q | | | | 26.0 q | 21.4 q |
| 11 | 16.6q | 25.6 q | 170 q | 23.6 q | 68.4 t | 10.9 q | 13.4 q | 17.0 q | 26.0 q | 68.4 q |
| 12 | 21.3q | 21.4q | 63.7 t | 21.6q | 21.5 q | 21.5 q | 21.6 q | 21.5 q | 21.5 q | 21.5 q |
| 13 | 31.9 t | 31.8 t | 32.1 t | 33.2 t | 33.0 t | 33.2 t | 33.2 t | 30.2 t | 30.2 t | 30.2 t |
| 14 | 61.4t | 61.7t | 62.2 t | 61.8t | 61.9 t | 61.8 t | 61.81 | 69.3 t | 69.3 t | 69.3 t |
| 15 | 13.7 q | 13.7 q | 13.8 q | 14.3q | 14.1 q | 14.2 q | 14.2 q | 14.0 q | 14.0 q | 14.1 q |
| 1' | | | | | | | | 104.6 d | 104.6 d | 104.6 d |
| 2' | | | | | | | | 75.3 d | 75.3 d | 75.3 d |
| 3' | | | | | | | | 78.3 d | 78.3 d | 78.3 d |
| 4' | | | | | | | | 71.7 d | 71.7 d | 71.7 d |
| 5' | | | | | | | | 78.1 d | 78.1 d | 78.1 d |
| 6' | | | | | | | | 62.8 t | 62.8 t | 62.8 t |

[a] Assignments were aided by a combination of COSY, HSQC, and HMBC experiments. [b] Measured in $CDCl_3$. [c] Carbon multiplicity.

**Example 3: Evaluation of cytotoxicity of pterosin compounds (MTT assay)**

In order to confirm that Compounds 2 to 11 of Example 2 exhibit toxic effects on cells, the following experiment was conducted.

As described in the experimental method, mouse embryo fibroblast cell-line (NIH3T3) as a normal cell and Mouse melanoma cell-line (B16F10) as a cancer cell were cultured in a 96-well plate, and each compound was added to the cell medium at each concentration and cultured for 48 hours, MTT was then administered, and the optical density was measured at 550 nm by using a microplate reader. The optical density is an index representing the number of surviving cells and calculated by the following formula, and reproducibility was confirmed through three experiments.

Cell proliferation rate (%) = OD550 (sample) / OD550 (control) X 100

Then, the LD50 (Lethal Dose 50) value was calculated based on the change in cell proliferation rate.

As a result, as shown in Table 3, the LD50 value of Compound 6 in cancer cells was the lowest at 522±25μM, the LD50 value of Compound 2 in normal cells was 3,110±130μM, and the LD50 value of Compound 3 in normal cells was 553±37 μM, the LD50 value of Compound 3 in cancer cells was found to be 618±71 μM. Except for this, the LD50 values of other compounds in normal cells and cancer cells were found to be greater than 5000 μM.

Therefore, it was confirmed that Compound 3 has weak cytotoxicity, Compound 6 has weak cytotoxicity only in cancer cells, and Compounds 2, 4, 5, 7 to 11 have little cytotoxicity in normal cells and cancer cells.

[Table 3]

| Cytotoxic effect of pterosin compounds | | |
|---|---|---|
| Compounds | $LD_{50}$ (μM) | |
| | Normal cell | Cancer cell |
| | NIH3T3 | B16F10 |
| Compound 2 (Pterosin B) | 3,1101130 | >5,000 |
| Compound 3 (Pterosin Z) | 553137 | 618171 |
| Compound 4 (Pterosin P) | >5,000 | >5,000 |
| Compound 5 (Pterosin D) | >5,000 | >5,000 |
| Compound 6 (Pterosin A) | >5,000 | 522125 |
| Compound 7 (Pterosin C *trans*) | >5,000 | >5,000 |
| Compound 8 (Pterosin C *cis*) | >5,000 | >5,000 |
| Compound 9 (Pterosin B) | >5,000 | >5,000 |
| Compound 10 (Pterosin Z) | >5,000 | >5,000 |
| Compound 11 (Pterosin A) | >5,000 | >5,000 |
| Compound 12 (Pterosin N) | >5,000 | >5,000 |

**Example 4: Rigid docking experiment for the cAMP binding site of PKA**

Experiments were carried out to predict PKA binding models of pterosin compounds or derivatives thereof.

For the experiment, Schrodingr's MAESTRO software was used, and the binding between the protein and the drug (pterosin A) was measured by using the Ligand docking and induced fit docking methods. The cAMP binding domains (CBDs) of the PKA regulatory subunit were two sites (i.e., CBD1 and CBD2) and binding to each of them were measured (see Fig. 3), and, as a result, docking scores at CBD1 and CDB2 were measured by -8.307 and -9.808, respectively, thereby confirming that protein-drug binding was very stable (see Table 4).

**[Table 4]**

| Docking Score | cAMP | (2S)-Pterosin A | Etc. |
|---|---|---|---|
| **CBD1** | -9.031 | -8.307 | The lower the docking score, the more stable the protein-drug binding. |
| **CBD2** | -8.376 | -9.808 | Docking score <-9: Excellent Docking score -8 to -7: Very good |

**Example 5: Western blot for measuring a CREB phosphorylation level**

As shown in FIG. 2, pterosin derivatives having a -OH group as a side chain at the bottom of the pentagonal ring of pterosin, such as pterosin A, C and D in neurons, significantly increased phosphodiesterase (PDE) at 10μM to levels above the hosphodiesterase (PDE) inhibitor amx as a positive control.

On the other hand, pterosin derivatives such as pterosin B, P and Z, which do not have a -OH group in the side chain at the bottom of the pentagonal ring of pterosin, did not increase CREB phosphorylation at all at 10 μM.

In astrocytes, which are connective tissue cells of the brain, both pterosin derivatives such as pterosin A, C and D, which have -OH groups in the side chains at the bottom of the pentagonal ring of pterosin, and pterosin derivatives free of -OH group in the side chain at the bottom of the pentagonal ring of pterosin such as pterosin B, P and Z had no effect on CREB phosphorylation.

Next, the effects of pterosin A, C, D and B on CREB, BDNF and trk phosphorylation in neurons were confirmed by Western blotting.

As a result, as shown in FIG. 8, when comparing pterosin derivatives having a -OH group in the side chain at the bottom of the pentagonal ring with pterosin B free of -OH group in the side chain, Pterosin A, C and D statistically significantly increased the phosphorylation of CREB through PKA activation. Accordingly, it was confirmed that the derivatives having a -OH group increase BDNF, which is a brain-derived neurotrophic factor that acts as one of the most important targets in cognitive disorders such that pterosin activates the PKA-CREB-BDNF signaling pathway, thereby

showing efficacy in alleviating learning and memory disorders.

**Example 6: Electrophysiology experiments**

Referring to the results of electrophysiological experiments in hippocampal slices, there was no particular change in normal synaptic transmission by the treatment of the test substance (FIG. 9). However, it was confirmed that hippocampus mossy fiber-CA1 LTP, a brain tissue responsible for long-term memory, was enhanced by pterosin compound treatment, and such effect was particularly strong with pterosin C and D (FIG. 10).

**Example 7: Neuron Proliferation, Differentiation and Regeneration Promoting Ability**

The abilities of pterosin compounds to promote nerve cell proliferation, differentiation and regeneration were evaluated.

As shown in FIG. 11, the length of nerve cells treated with pterosin D was significantly longer than that of the control group, confirming that the effect of promoting nerve cell proliferation and differentiation appeared.

**Example 8: Evaluation of blood-brain barrier permeability**

The blood-brain barrier permeability of Pterosin D, one of the compounds showing the most excellent activities in the above-described examples, was evaluated according to the method below. Progesterone was used as a positive control and ranitidine was used as a negative control:

(1) A donor buffer at pH 7.4 was dispensed to a deep well plate and mixed with a test substance.
(2) The donor buffer was dispensed into a U.V plate by 150ul per well, and the optical density was measured: blank value.
(3) The mixture was dispensed in the deep well plate into the U.V plate by 150ul per well, and then the optical density was measured: blank value.
(4) 200ul of the mixture in step 1) was dispensed into the donor plate of the Stirwell PAMPA sandwich.
(5) The membrane of the acceptor plate of the Stirwell PAMPA sandwich was coated with 5 ul of BBB-1-lipid.
(6) A brain sink buffer was dispensed into each well on the acceptor plate by 200 ul.
(7) The acceptor plate and donor plate were combined and incubated at 25°C for 4 hours.
(8) After 4 hours of incubation, the acceptor plate and donor plate were separated, and 150 μl each was transferred to a UV plate, and the optical density was measured.

Permeability evaluation criteria are shown in Table 5 below:

[Table 5]

| Permeability (BBB permeability) | | CNS +/- (spreadability to CNS) | |
|---|---|---|---|
| $P_e$ ($10^{-6}$cm/sec) | BCS code | $P_e$ ($10^{-6}$ cm/sec) | BCS code |
| >0.4 | High | >10 | CNS+ |
| <0.4 | Low | <10 | CNS- |

The results of evaluating the BBB permeability of Pterosin D and the control group are shown in Table 6 below:

[Table 6]

| Name | pH | $P_e$ ($10^{-6}$cm/sec) | | | | | BCS code |
|---|---|---|---|---|---|---|---|
| | | 1st | 2nd | 3rd | Average | SD | |
| Progesterone (50 μM) | 7.4 | 29.28 | 28.45 | 23.77 | 28.84 | 0.42 | High (CNS+) |
| Ranitidine (50 μM) | 7.4 | 0 | 0 | 0 | 0 | - | Low (CNS-) |
| Pterosin D (12.5 μM) | 7.4 | 19.74 | 20.20 | 22.73 | 20.89 | 1.61 | High (CNS+) |
| - $P_e$: Effective permeability coefficients | | | | | | | |

## INDUSTRIAL APPLICABILITY

As described above, the composition for preventing or treating diseases comprising the pterosin compound and derivatives thereof as an active ingredient exhibits provided by the present invention has the effect of activating PKA and CREB and, thus, can be used for development of a therapeutic agent for depression, drug addiction, Rubinstein Taybi syndrome, insomnia, delayed sleep syndrome, advanced sleep phase syndrome, cerebral palsy, autism, attention deficit hyperactivity disorder (ADHD) and paraplegia, or food for improving the diseases. Therefore, the industrial applicability of the composition of the present invention is very excellent.

## Claims

1. A pharmaceutical composition for the prevention or treatment of neuropsychiatric diseases, comprising a compound defined by the following formula 1, an isomer thereof, or a pharmaceutically acceptable salt thereof as an active ingredient:

[Formula 1]

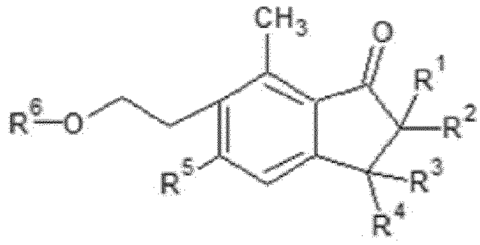

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

2. The pharmaceutical composition of claim 1, wherein at least one of R1 to R4 is OH or an alcohol having 1 to 4 carbon atoms.

3. The pharmaceutical composition of claim 1, wherein any one of R1 to R4 is OH or an alcohol having 1 to 4 carbon atoms, and R6 is hydrogen.

4. The pharmaceutical composition of claim 1, wherein the compound defined by formula 1 is selected from the group consisting of pterosin A, pterosin C, pterosin D and pterosin N.

5. The pharmaceutical composition of claim 1, wherein the compound defined by formula 1 activates Protein kinase A (PKA) .

6. The pharmaceutical composition of claim 1, wherein the compound defined by formula 1 increases CREB phosphorylation.

7. The pharmaceutical composition of claim 1, wherein the compound defined by formula 1 is extracted from bracken.

8. The pharmaceutical composition of claim 1, wherein the neuropsychiatric diseases are selected from the group consisting of Huntington's disease, cerebral palsy, autism, attention deficit hyperactivity disorders (ADHD), paraplegia, depression, drug addiction, Rubinstein Taybi syndrome, insomnia, delayed sleep syndrome and advanced sleep phase syndrome.

9. A food composition for the prevention or improvement of neuropsychiatric diseases, comprising a compound defined by the following formula 1, an isomer thereof, or a pharmaceutically acceptable salt thereof as an active ingredient:

[Formula 1]

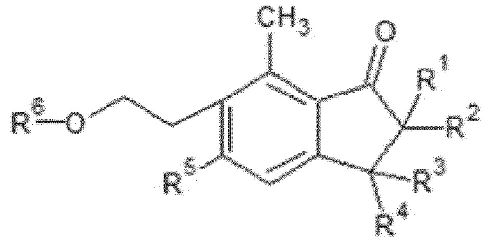

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

**10.** Use of a compound defined by the following formula 1, an isomer thereof or a pharmaceutically acceptable salt thereof for preparing a preparation for use in the treatment of neuropsychiatric diseases:

[Formula 1]

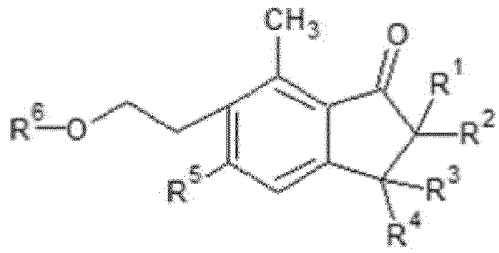

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

**11.** The use of claim 10, wherein the compound defined by formula 1 is extracted from bracken.

**12.** The use of claim 10, wherein the neuropsychiatric diseases are selected from the group consisting of Huntington's disease, cerebral palsy, autism, attention deficit hyperactivity disorders (ADHD), paraplegia, depression, drug addiction, Rubinstein Taybi syndrome, insomnia, delayed sleep syndrome and advanced sleep phase syndrome.

**13.** A method for treating neuropsychiatric diseases, comprising administering an effective amount of a composition comprising the compound defined by the following formula 1, an isomer thereof or a pharmaceutically acceptable salt thereof to a subject in need thereof:

[Formula 1]

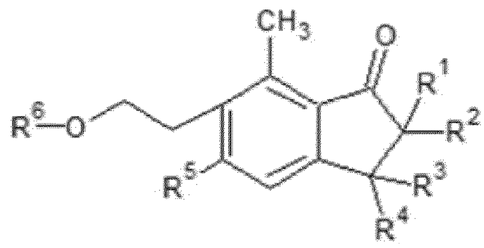

wherein
R1, R2, R3 and R4 each independently are H, OH, or an alkyl or alcohol having 1 to 4 carbon atoms; R5 and R6 each independently are H, OH, alkyl, alkenyl, alkynyl, alcohol, carboxyl, ether, cycloalkyl, heterocycloalkyl or glucose.

**14.** The method of claim 13, wherein the compound defined by formula 1 is extracted from bracken.

**15.** The method of claim 13, wherein the neuropsychiatric diseases are selected from the group consisting of Huntington's disease, cerebral palsy, autism, attention deficit hyperactivity disorders (ADHD), paraplegia, depression, drug addiction, Rubinstein Taybi syndrome, insomnia, delayed sleep syndrome and advanced sleep phase syndrome.

FIG. 1

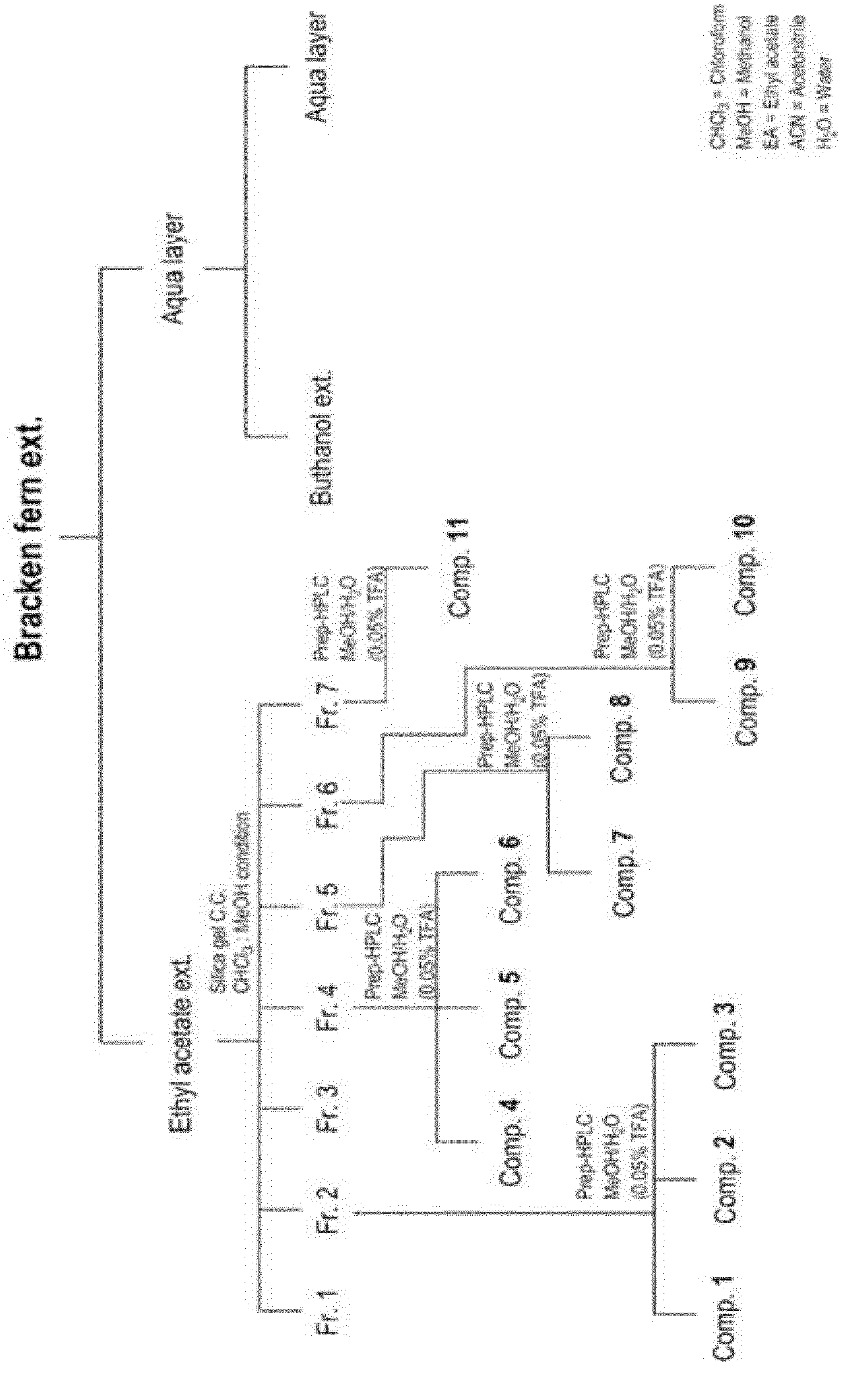
Bracken fern ext.
Ethyl acetate ext.
Aqua layer
Buthanol ext.
Aqua layer
Silica gel C.C.
$CHCl_3$ : MeOH condition
Fr. 1
Fr. 2
Fr. 3
Fr. 4
Fr. 5
Fr. 6
Fr. 7
Prep-HPLC
MeOH/$H_2O$
(0.05% TFA)
Comp. 1
Comp. 2
Comp. 3
Comp. 4
Comp. 5
Comp. 6
Comp. 7
Comp. 8
Comp. 9
Comp. 10
Comp. 11
$CHCl_3$ = Chloroform
MeOH = Methanol
EA = Ethyl acetate
ACN = Acetonitrile
$H_2O$ = Water

FIG. 2

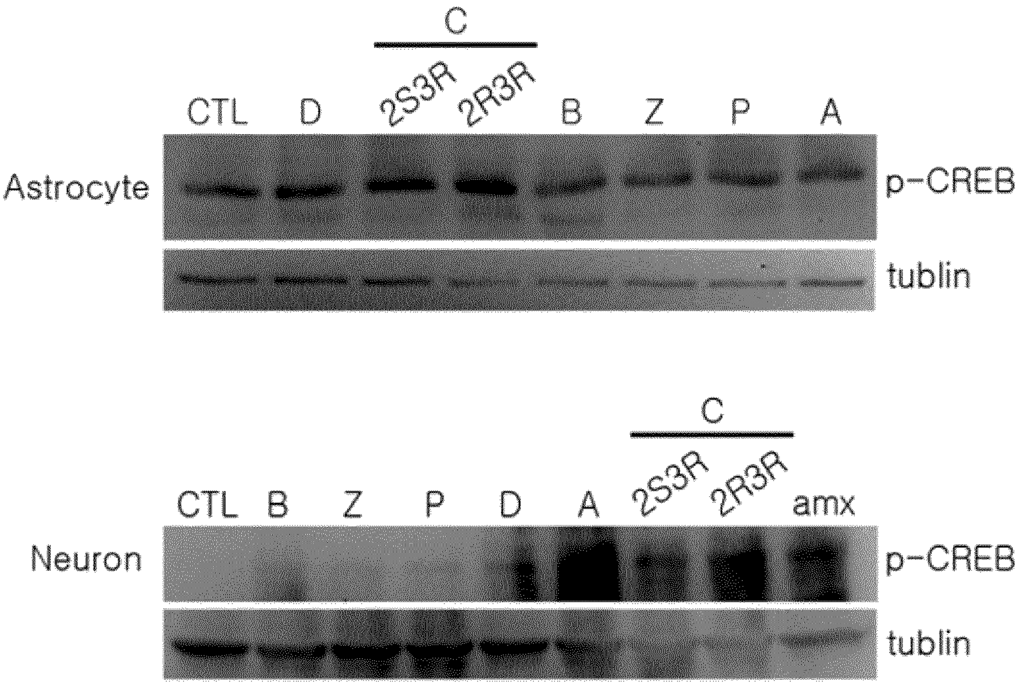
C
CTL
D
2S3R
2R3R
B
Z
P
A
Astrocyte
p-CREB
tublin
C
CTL
B
Z
P
D
A
2S3R
2R3R
amx
Neuron
p-CREB
tublin

FIG. 3

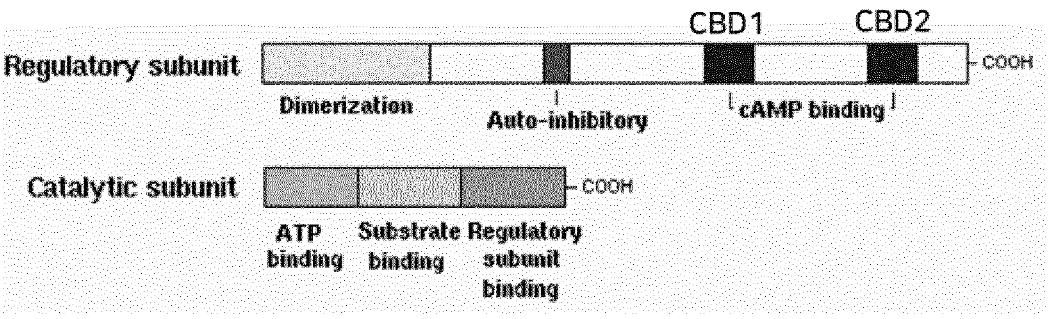
CBD1
CBD2
Regulatory subunit
COOH
Dimerization
Auto-inhibitory
cAMP binding
Catalytic subunit
COOH
ATP binding
Substrate binding
Regulatory subunit binding

FIG. 4

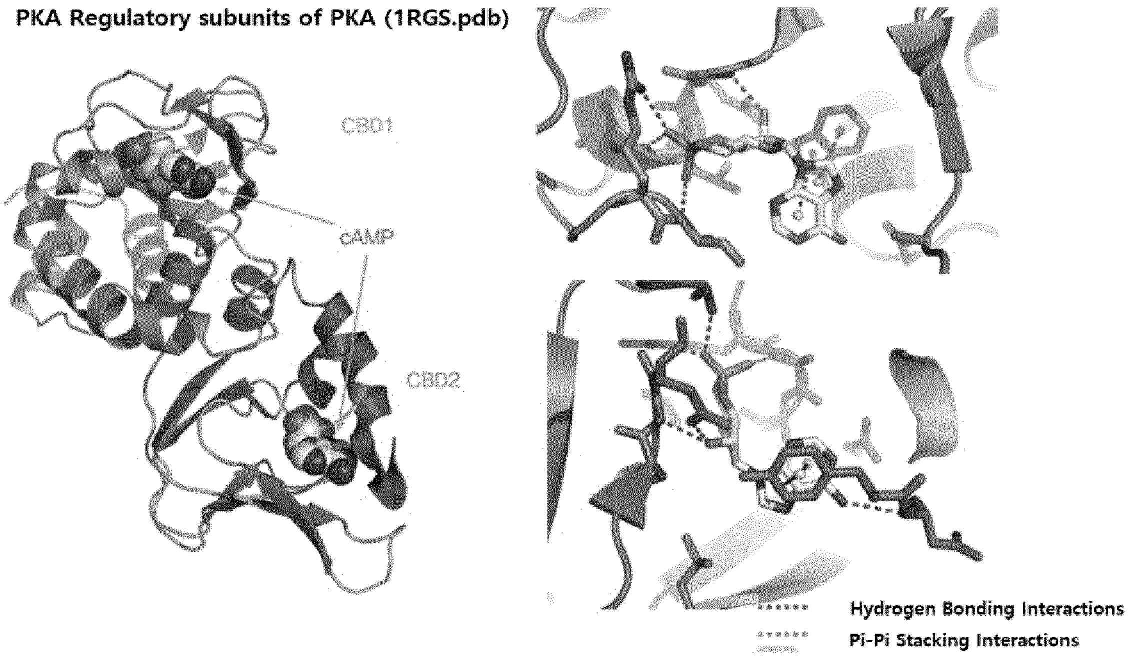
PKA Regulatory subunits of PKA (1RGS.pdb)
CBD1
cAMP
CBD2
Hydrogen Bonding Interactions
Pi-Pi Stacking Interactions

FIG. 5

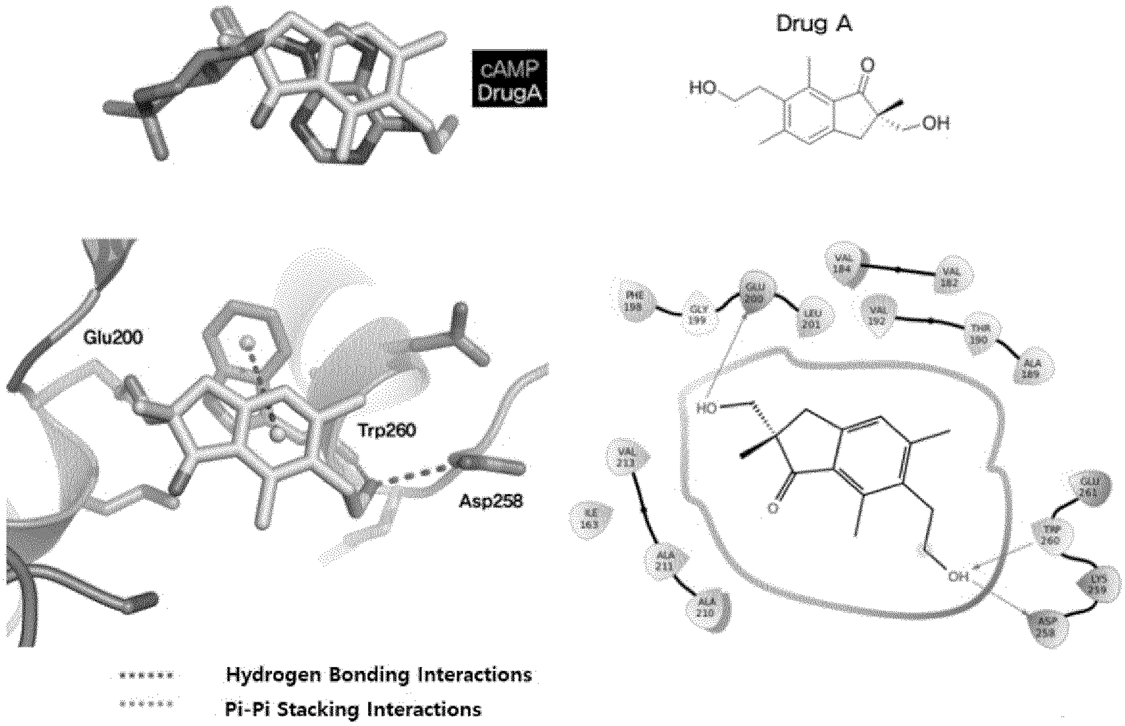
cAMP
DrugA
Drug A
Glu200
Trp260
Asp258
Hydrogen Bonding Interactions
Pi-Pi Stacking Interactions

FIG. 6

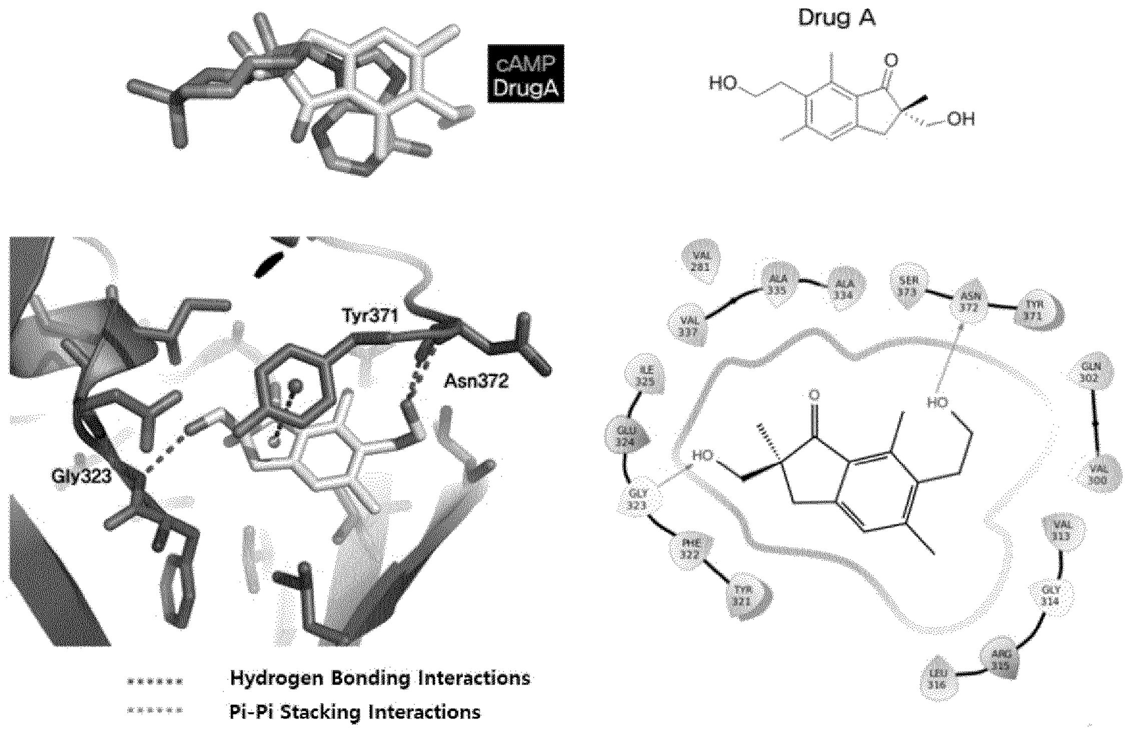
cAMP
DrugA
Drug A
Tyr371
Asn372
Gly323
VAL 281
ALA 335
ALA 334
SER 373
ASN 372
TYR 371
VAL 337
GLN 302
ILE 325
GLU 324
VAL 300
GLY 323
VAL 313
PHE 322
GLY 314
TYR 321
ARG 315
LEU 316
Hydrogen Bonding Interactions
Pi-Pi Stacking Interactions

FIG. 7

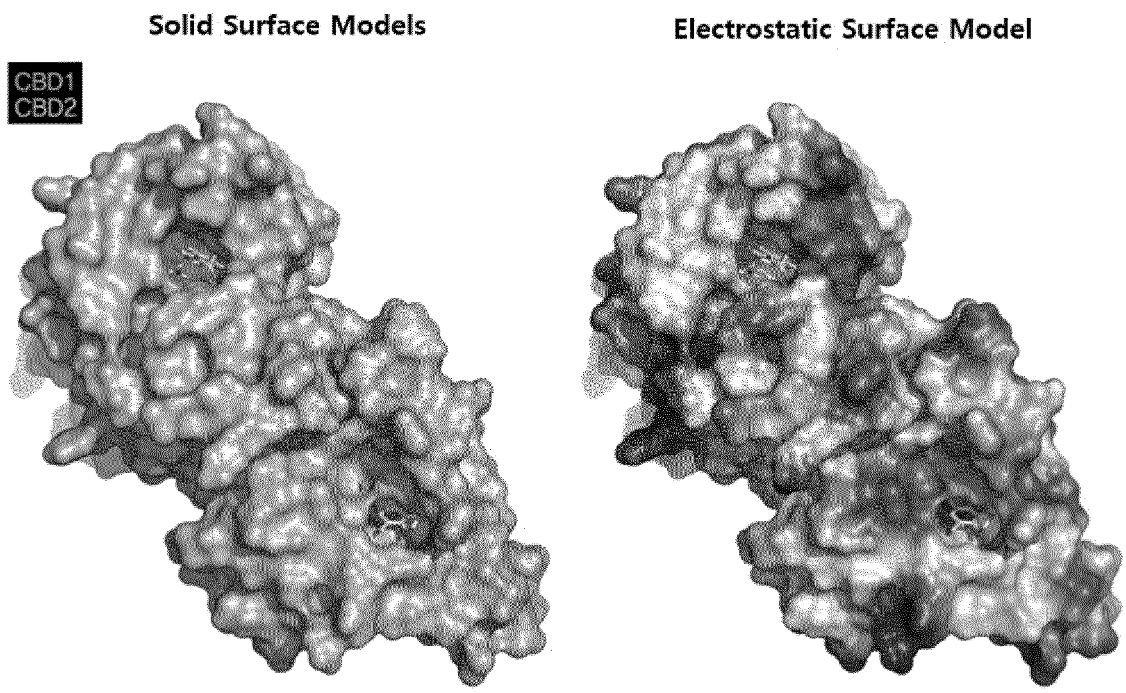
Solid Surface Models
Electrostatic Surface Model
CBD1
CBD2

FIG. 8

# Western blots: PKA, p-CREB. p-Trk, BDNF

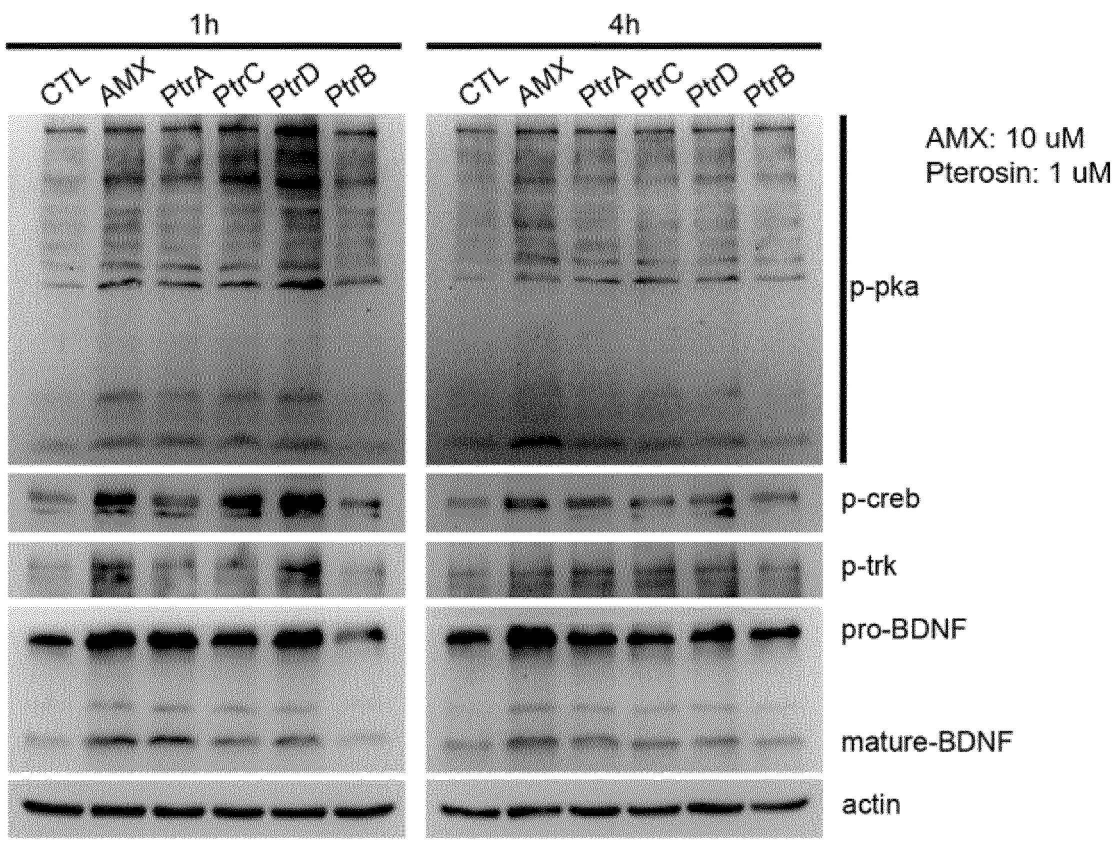

FIG. 9

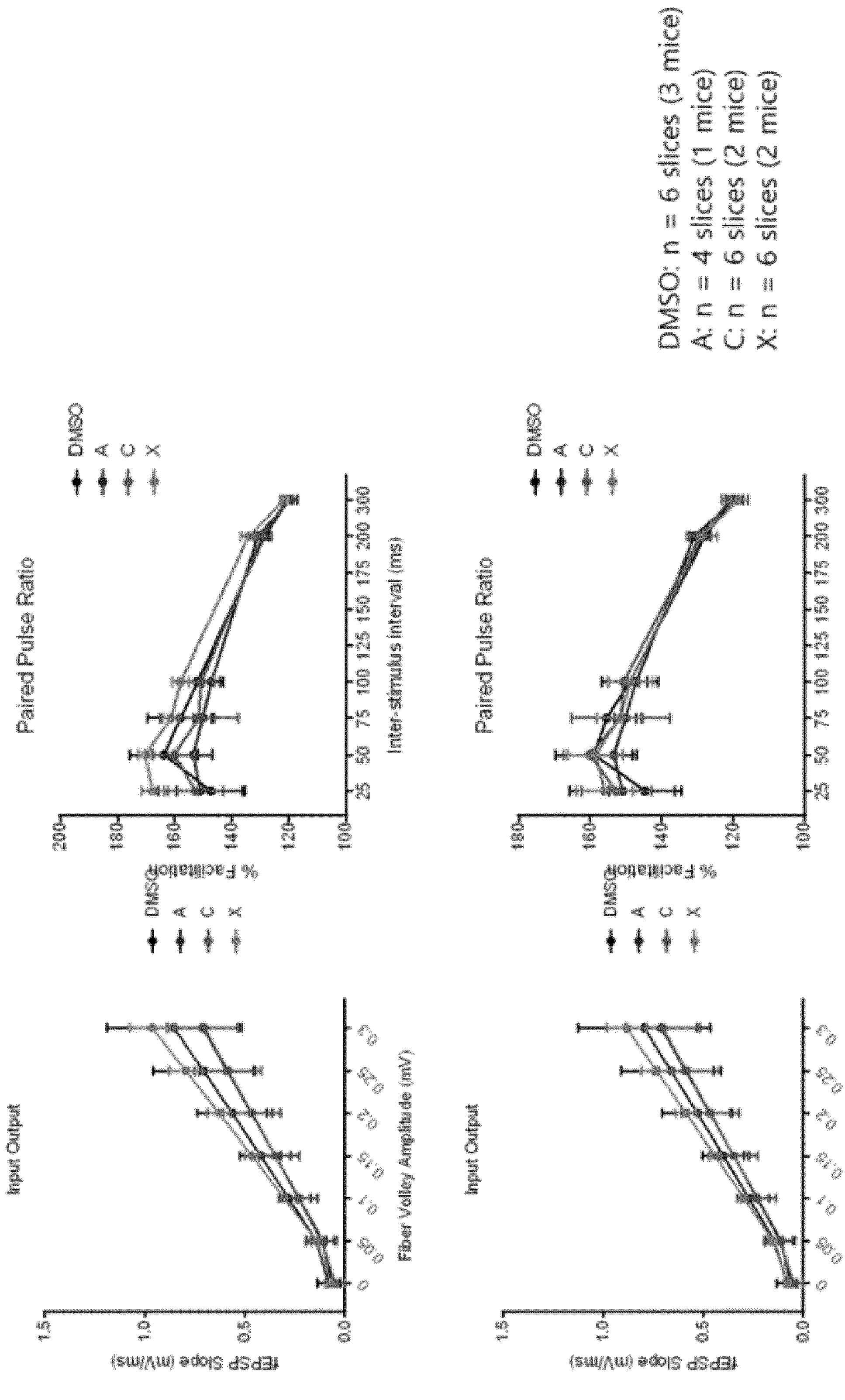
Input Output
fEPSP Slope (mV/ms)
Fiber Volley Amplitude (mV)
Paired Pulse Ratio
% Facilitation
Inter-stimulus interval (ms)
DMSO
A
C
X
Input Output
fEPSP Slope (mV/ms)
Fiber Volley Amplitude (mV)
Paired Pulse Ratio
% Facilitation
Inter-stimulus interval (ms)
DMSO
A
C
X
DMSO: n = 6 slices (3 mice)
A: n = 4 slices (1 mice)
C: n = 6 slices (2 mice)
X: n = 6 slices (2 mice)

FIG. 10

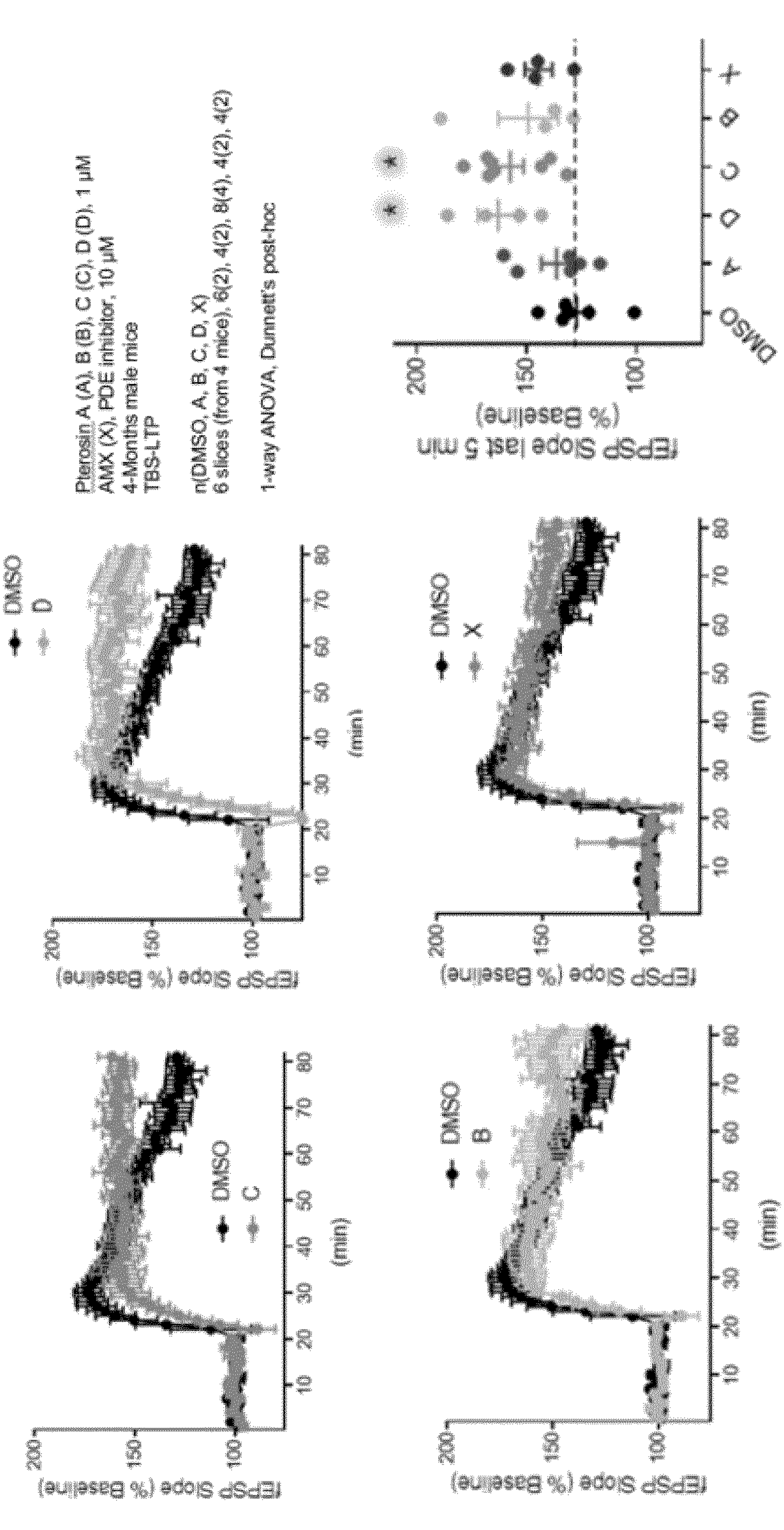
Pterosin A (A), B (B), C (C), D (D), 1 μM
AMX (X), PDE inhibitor, 10 μM
4-Months male mice
TBS-LTP
n(DMSO, A, B, C, D, X)
6 slices (from 4 mice), 6(2), 4(2), 8(4), 4(2), 4(2)
1-way ANOVA, Dunnett's post-hoc
fEPSP Slope (% Baseline)
fEPSP Slope last 5 min (% Baseline)
(min)
DMSO
C
D
B
X
A
*

FIG. 11

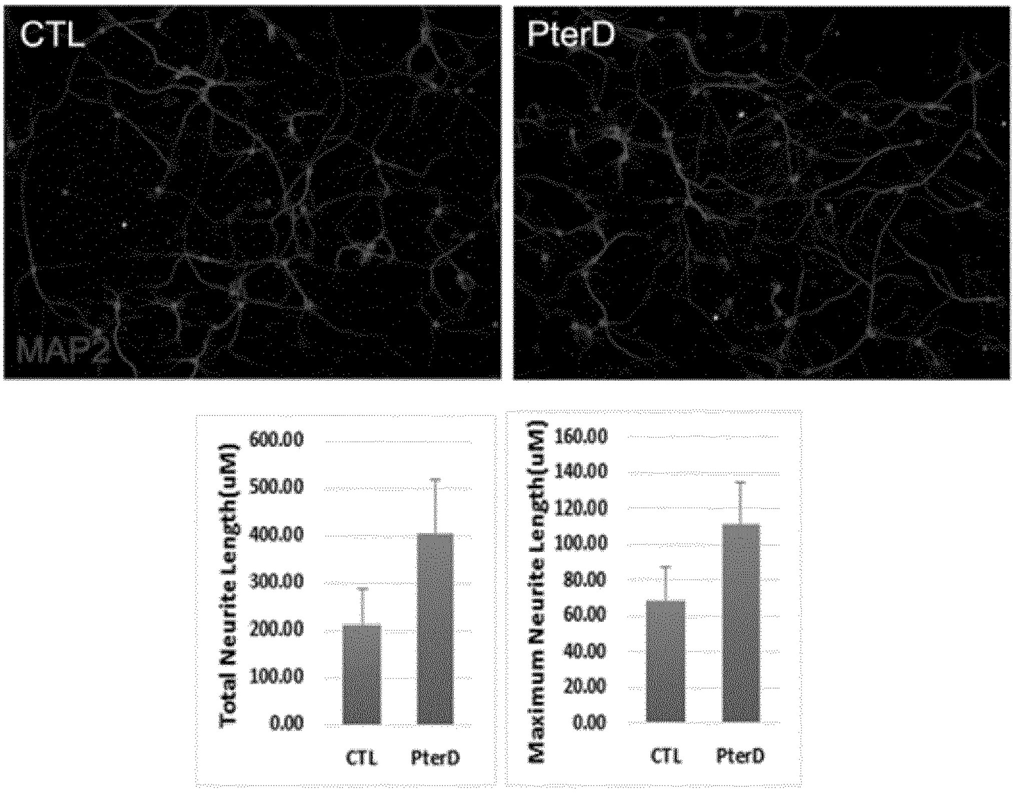
CTL
MAP2
PterD
Total Neurite Length(uM)
600.00
500.00
400.00
300.00
200.00
100.00
0.00
CTL
PterD
Maximum Neurite Length(uM)
160.00
140.00
120.00
100.00
80.00
60.00
40.00
20.00
0.00
CTL
PterD

**INTERNATIONAL SEARCH REPORT**

International application No. **PCT/KR2021/018989**

**A. CLASSIFICATION OF SUBJECT MATTER**

**A61K 31/122**(2006.01)i; **A61K 36/11**(2006.01)i; **A61P 25/00**(2006.01)i; **A23L 33/10**(2016.01)i

According to International Patent Classification (IPC) or to both national classification and IPC

**B. FIELDS SEARCHED**

Minimum documentation searched (classification system followed by classification symbols)

A61K 31/122(2006.01); A23L 33/10(2016.01); A61K 31/12(2006.01); A61K 31/404(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal), STN (Registry, CAplus) & keywords: 프테로신(pterosin), 신경정신성 질환(neuropsychiatric disease), PKA(protein kinase A, cAMP-dependent protein kinase)

**C. DOCUMENTS CONSIDERED TO BE RELEVANT**

| Category* | Citation of document, with indication, where appropriate, of the relevant passages | Relevant to claim No. |
|---|---|---|
| DX | KR 10-2018-0138555 A (GYEONGGIDO BUSINESS & SCIENCE ACCELERATOR et al.) 31 December 2018 (2018-12-31)<br>See claims 1-5 and 7-9; paragraphs [0090]-[0092] and [0096]; and table 1. | 1-12 |
| A | KR 10-2011-0125642 A (TAIPEI MEDICAL UNIVERSITY et al.) 21 November 2011 (2011-11-21)<br>See claims 1, 12 and 13; and paragraphs [0038] and [0039]. | 1-12 |
| A | ITOH, Y. et al. Salt-inducible kinase 3 signaling is important for the gluconeogenic programs in mouse hepatocytes. Journal of biological chemistry. 2015, vol. 290, no. 29, pp. 17879-17893.<br>See abstract; and figure 3. | 1-12 |
| A | ITOH, Y. et al. Pterosin B has multiple targets in gluconeogenic programs, including coenzyme Q in ROR α–SRC2 signaling. Biochemical and Biophysical Research Communications. 2016, vol. 473, pp. 415-420.<br>See abstract. | 1-12 |
| A | CHEN, C. Y. et al. Chemical constituents analysis and antidiabetic activity validation of four fern species from Taiwan. International journal of molecular sciences. 2015, vol. 16, pp. 2497-2516.<br>See abstract. | 1-12 |

☐ Further documents are listed in the continuation of Box C. ☑ See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **18 March 2022** | **18 March 2022** |
| Name and mailing address of the ISA/KR<br>**Korean Intellectual Property Office**<br>**Government Complex-Daejeon Building 4, 189 Cheongsa-ro, Seo-gu, Daejeon 35208**<br>Facsimile No. **+82-42-481-8578** | Authorized officer<br><br>Telephone No. |

Form PCT/ISA/210 (second sheet) (July 2019)

**INTERNATIONAL SEARCH REPORT**

International application No.
**PCT/KR2021/018989**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-15**
because they relate to subject matter not required to be searched by this Authority, namely:

   Claims 13-15 pertain to a method for treatment of the human body by surgery or therapy (PCT Article 17(2)(a)(i) and PCT Rule 39.1(iv)).

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

Form PCT/ISA/210 (continuation of first sheet) (July 2019)

**INTERNATIONAL SEARCH REPORT**
**Information on patent family members**

International application No.
**PCT/KR2021/018989**

| Patent document cited in search report | Publication date (day/month/year) | Patent family member(s) | Publication date (day/month/year) |
|---|---|---|---|
| KR 10-2018-0138555 A | 31 December 2018 | CN 111225664 A | 02 June 2020 |
| | | EP 3639817 A1 | 22 April 2020 |
| | | KR 10-1934527 B1 | 02 January 2019 |
| | | KR 10-2018-0137265 A | 27 December 2018 |
| | | KR 10-2085358 B1 | 05 March 2020 |
| | | WO 2018-230776 A1 | 20 December 2018 |
| KR 10-2011-0125642 A | 21 November 2011 | AU 2010-206549 A1 | 11 August 2011 |
| | | AU 2010-206549 A9 | 25 August 2016 |
| | | AU 2010-206549 B2 | 25 August 2016 |
| | | CA 2749931 A1 | 29 July 2010 |
| | | CA 2749931 C | 06 March 2018 |
| | | CN 102333528 A | 25 January 2012 |
| | | CN 102333528 B | 18 December 2013 |
| | | EP 2389171 A2 | 30 November 2011 |
| | | EP 2389171 B1 | 30 March 2016 |
| | | JP 2012-515801 A | 12 July 2012 |
| | | JP 5640019 B2 | 10 December 2014 |
| | | KR 10-1746867 B1 | 14 June 2017 |
| | | NZ 594236 A | 29 August 2014 |
| | | TW 201039836 A | 16 November 2010 |
| | | TW I522109 B | 21 February 2016 |
| | | US 2010-0190732 A1 | 29 July 2010 |
| | | US 8633252 B2 | 21 January 2014 |
| | | WO 2010-085811 A2 | 29 July 2010 |
| | | WO 2010-085811 A9 | 04 November 2010 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- KR 20200174821
- KR 102085358


### Non-patent literature cited in the description


- **RUSH et al.** *Am. J. Psychiatry,* 2006, vol. 163, 1905
- **TAKAHASHI et al.** *Phytother. Res,* 2004, vol. 18, 573
- **SHERIDAN et al.** *Planta Med.,* 1999, vol. 65, 271
- **NAGAO et al.** *Mutation Research,* 1989, vol. 215, 173
- **MURAKAMI et al.** *Chem. Pharm. Bull,* 1976, vol. 24, 2241
- **KURAISHI et al.** *Chem. Pharm. Bull,* 1985, vol. 33, 2305J
- **BANERJI.** *Tetrahedron Letters,* 1974, vol. 15, 1369
- **HAYASHI et al.** *Tetrahedron Letters,* 1991, vol. 33, 2509
- **MCMORRIS et al.** *J.Org. Chem,* 1992, vol. 57, 6876
- **R. LAROCK.** Comprehensive Organic Transformations. VCH Publishers, 1989
- **T. W. GREENE ; P. G. M. WUTS.** Protective Groups in Organic Synthesis. John Wiley and Sons, 1999
- **L. FIESER ; M. FIESER.** Fieser and Fieser's Reagents for Organic Synthesis. John Wileyand Sons, 1994
- Encyclopedia of Reagents for Organic Synthesis. John Wiley and Sons, 1995
- **BELMAKER, R. H. ; AGAM, GALILA.** Major depressive disorder. *New England Journal of Medicine,* 2008, vol. 358 (1), 55-68
- **BLENDY, J. A.** The role of CREB in depression and antidepressant treatment. *Biol Psychiatry,* 2006, vol. 59 (12), 1144-50
- **GINTY, D. D. ; KORNHAUSER, J. M ; THOMPSON, M. A ; BADING, H ; MAYO, K. E ; TAKAHASHI, J. S ; GREENBERG, M. E.** Regulation of CREB phosphorylation in the suprachiasmatic nucleus by light and a circadian clock. *Science,* 09 April 1993, vol. 260 (5105), ISSN ISSN 0036-8075, 238-241
- **GAU, DANIEL ; LEMBERGER, THOMAS ; VON GALL ; CHARLOTTE; KRETZ, OLIVER ; LE MINH, NGUYET ; GASS, PETER ; SCHMID, WOLFGANG ; SCHIBLER, UELI ; KORF, HORST W.** Phosphorylation of CREB Ser142 Regulates Light-Induced Phase Shifts of the Circadian Clock. *Neuron,* 11 April 2002, vol. 34 (2), 245-253